# EUROPEAN PATENT SPECIFICATION

(11) **EP 1 331 874 B1**
(45) Date of publication and mention of the grant of the patent: **05.08.2009**
(21) Application number: 01946393.4
(22) Date of filing: 15.06.2001
(51) Int. Cl.: A61B 5/00

(54) **A HEALTH OUTCOMES AND DISEASE MANAGEMENT NETWORK FOR PROVIDING IMPROVED PATIENT CARE**
GESUNDHEITS- UND KRANKHEITSVERWALTUNGSSYSTEM ZUR VERBESSERTEN PATIENTENVERSORGUNG
RESEAU DE GESTION DE BILANS DE SANTE ET D'ETATS PATHOLOGIQUES PERMETTANT D'AMELIORER LES SOINS APPORTES AUX PATIENTS

(30) Priority: 30.06.2000 US 215254 P
(43) Date of publication of application: 06.08.2003
(73) Proprietor: Becton Dickinson and Company, Franklin Lakes, New Jersey 07417 (US); Duke University, Durham, NC 27710 (US)
(72) Inventor: VONK, Glenn, Fuguay-Varina, NC 27526 (US); FRANTZ, Ann, Pontiac, MI 48340 (US); WHELLAN, David, Durham, NC 27705 (US); O'CONNOR, Christopher, Durham, NC 27707 (US); GOLDMAN, George, Boonton Township, NJ 07005 (US)
(74) Representative: Ruffles, Graham Keith
(86) International application number: PCT/US2001/019198
(87) International publication number: WO 2002/002004

(56) References cited:
- FR-A- 2 760 962
- US-A- 5 301 105
- US-A- 5 517 405
- US-A- 5 549 117
- US-A- 5 851 186
- US-A- 5 867 821
- US-A- 5 911 687
- US-A- 5 937 387
- US-A- 5 960 403
- US-A- 6 014 631

## Description

The present application claims benefit under 35 U.S.C. § 119(e) of a U.S. provisional application of Glenn P. Vonk, Ann K. Frantz, David J. Whellan, Christopher M. O'Connor and George Goldman entitled "A Health Outcomes and Disease Management Network and Related Method for Providing Improved Patient Care", Serial No. 60/215,254, filed June 29, 2000.

A portion of the disclosure of this patent document contains material which is subject to copyright protection. The copyright owner has no objection to the facsimile reproduction by any one of the patent document or the patent disclosure, as it appears in the Patent and Trademark Office patent file or records, but otherwise reserves all copyright rights whatsoever.

### BACKGROUND OF THE INVENTION

### Field of the Invention:

The present invention relates to a health outcomes and disease management network for providing improved healthcare. More particularly, the present invention relates to a network of healthcare managers and healthcare providers who interactively cooperate with patients to monitor and evaluate patient status to provide the most appropriate treatment for the patients in the most cost-effective manner, thus improving overall healthcare. The health outcomes and disease management network consists of both local networks located within a healthcare provider organization, and a meta-network which links the local networks to a metaservice. Meta-services aggregate data from local networks, process the data to reveal population trends and outcomes, and provide rapid feedback and information on the best medical/economic practices to the local networks.

### Description of the Related Art:

Modem healthcare and disease management is becoming more complex due to the many different options that are becoming available for providing short-term and long-term patient treatment, as well as the many different types of payment and insurance programs in existence. In evaluating all the different options to arrive at the most suitable, the interests of all "stakeholders" involved should be taken into consideration.

The various stakeholders involved in healthcare may be broadly classified into three groups: payors, providers (or care teams), and patients. Payors generally address concerns associated with increasing financial pressures of healthcare. Payors tend to be skeptical about new concepts since their profit margins are generally at risk and declining.

Payors include traditional fee for service insurance companies, Health Maintenance Organizations (HMOs), Physician Provider Organizations (PPOs), and Integrated Delivery Networks (IDNs). The government is also a payor, and is continually looking for new ways to reduce the cost of care delivery in order to control escalating medical expenditures. Government payors are particularly important since they pay for the most care and set precedents for payment.

Providers include hospitals, nurses, case managers, social workers, doctors, and many others in contact with patients. Providers are largely concerned with efficiency and quality of the care delivered, and frequently complain that they have little time for preventive interventions since they have to limit their attention to acute cases. This sub-optimization in care delivery is universally appreciated, but little has been done to bring a more strategic perspective to the providers' world.

Additionally, providers are often skeptical that these disease management initiatives might limit their flexibility to manage patients. Often, these initiatives are designed to bring additional cost controls to bear on provider organizations. These controls further reduce revenue for providers and drive the provider to a lower standard of care in order to increase patient volume and maintain income. Providers seeking good clinical outcomes for their patients are often frustrated by these situations.

Patients, on the other hand, are concerned with living a long life, having good care and good quality of life at an affordable price. Patients frequently complain of poor service, such as long waits, limited access to important information, and confusing billing, to name a few. Patients therefore often grow weary of endless repeated questions and being treated as a "member of plan A" rather than a person. Patients generally want to be in control of their own care. Few provider organizations inquire about the patient's goals even though the individual outcomes desired may vary considerably.

Related to patient is the patient's family. Often, a spouse, other immediate family member or significant other provides substantial support to the patient. These individuals may be granted pre-approval, in consultation with the patient, to access certain elements of the disease management network. For example, a family member may obtain educational information, information on the patient's status, access a chat session, and interact with the healthcare manager to provide effective local support to the patient.

Payors, providers, and patients are generally unsatisfied with healthcare services prevalent today.

Methods commonly referred to as "disease management" promise to address the diverse needs of the stakeholders, but have not delivered on these promises due to lack of sufficient integration. Present disease management companies may be classified into either service or device suppliers. Service suppliers have demonstrated that intensive patient management does significantly improve clinical outcomes. However, the cost of this intensive intervention by highly trained personnel has been unsustainable.

In response, certain of these companies have moved from a relatively high cost of home based or local delivery to a centralized, less intensive approach relying predominately on remote nurses interacting with patients by telephone. Under these circumstances, the care delivered has not been adequate to generate equivalent clinical outcomes. Further, these remote organizations lack credibility among local providers. Service oriented disease management companies have not succeeded broadly for any disease state.

Device oriented disease management companies appreciate the fact that remote service suppliers have been unable to effectively monitor patients at remote locations. These businesses seek to provide this monitoring using remote devices and a data network to transfer information about patient status to the providers. Examples of device oriented disease management systems are described in published International Patent Application No. WO 99/04043 of Abbott Laboratories, in published International Patent Application No. WO 99/46718 of Healthware Corporation, and in U.S. Patent No. 5,987,519.

Device oriented disease management companies have not succeeded broadly in the marketplace for several reasons. First, the devices are generally expensive and difficult to use. Second, the receipt of raw patient information presents a liability to the provider. The provider must evaluate this information in real time or risk litigation in the event that this information provided early warning of a significant health event that was not acted upon in time. Both device and service oriented companies have failed to justify their costs to healthcare organizations over time.

Frequently, clinical outcomes are presented for small numbers of patients over short time periods to establish program credibility. While clinical outcomes may look attractive, payors and providers are not generally convinced by these studies. Generally, there is minimal description of the necessary expenditures to deliver these results. Further, no information has been provided on the efficacy of any program to a number of health systems. Payors question if it will work in their venue. This incredulity has led to business models which reduce the risk of adoption by healthcare organizations through risk sharing.

Disease management companies may go at risk for sub-populations within provider organizations. The first phase of these arrangements allows the supplier to recover the investment needed to install the system. The second phase allows some split of the savings between provider, payor, and supplier. These arrangements sound attractive, but are difficult to administer since the supplier depends on the healthcare organizations to share the economic and clinical data required to administer the split. Also, there are significant cultural barriers for provider organizations to "carve out" populations to external suppliers. These barriers include the tacit admission that the provider organization cannot provide the care for the population and the concern that doctor - patient relationships will suffer.

A third difficulty with present disease management suppliers concerns poor definition of the optimal customer. Early disease management suppliers marketed systems to provider organizations. These suppliers succeeded only in very limited venues under extreme cost pressure or capitated reimbursement. Generally, provider organizations risk declining utilization when effective disease management is incorporated into healthcare delivery and are dis-incented to participate. Disease management has also been marketed to payor organizations with similar results. While some payors appreciate the potential of disease management they do not provide care. No matter how good the disease management system is, payors often lack influence to change care paradigms among providers. Neither can payors understand the subtle real and cultural issues which play when disease management is incorporated into the provider system.

Certain disease management models rely on patients to self-pay for care. However, most patients are reluctant to incur significant out of pocket expenses for healthcare. Yet another model seeks to market disease management to physicians. These models have limited financial potential and often are a financial liability to individual providers due to increased time requirements and lack of reimbursement for regular disease management consultations.

A fourth difficulty with disease management suppliers concerns their inability to encourage healthy behaviors. Most disease management programs use disease-specific population-based practice guidelines to manage high risk patient populations. These guidelines describe well defined patient and provider activities which improve clinical outcomes. Performance tracking against guidelines is often required to achieve accreditation as a center of excellence. However, performance tracking is costly and often not implemented even when the provider organization complies with accepted guidelines. Provider organizations which fail to demonstrate performance against accepted guidelines may risk loss of professional accreditation and erosion of goodwill. Further, provider organizations lack expertise to influence both individual providers and patients to adopt healthy behaviors suggested by the guidelines. What these organizations uniformly lack are tools to change behaviors. Performance benchmarking is one of the more useful tools for encouraging superior behaviors.

A fifth problem concerns the progress of medical knowledge, which rapidly obsoletes established practice. Medical practice is justifiably conservative. New therapies are often greeted with "healthy" skepticism until proven to be valuable across a wide diversity of unique patient concerns and comorbidities. However, the development of data driven and evidence based medicine will be greatly accelerated by telecommunications. It is expected that this information will, in turn, accelerate both the rate of change and the complexity of medical best practices. Providers will find it necessary to formulate integrated treatment plans which process personal genomic information, monitoring, assessments, pharmacogenomics, psychosocial evaluations, value, and other specialized information in the light of the patient's own expressed wishes and needs. Guidelines will become customizable to specific patient issues. Effective communication of improved guidelines to providers will be required to achieve the promised benefits. Ultimately, providers will desire tools to assist with the many complexities of customized best practices.

Accordingly, a need exists for a health outcomes and disease management network capable of providing optimum patient care in the most efficient and cost-effective manner, and which satisfies patients, providers and payors.

Patent number US 5,960,403 discloses a system as defined by the preamble of claim 1.

### SUMMARY OF THE INVENTION

The present invention is defined by the appended claims.

An objective of the present invention is to establish a health outcomes and disease management network which is capable of providing optimum patient care in the most efficient and cost-effective manner, and which satisfies patients, providers and payors.

Another objective of the present invention is to provide a healthcare management network that enables care managers and care providers to interact with patients to improve patient treatment, as well as overall healthcare for existing and new patients, while also taking issues pertaining to payors into consideration.

A further objective of the present invention is to provide a healthcare and disease management network that enables patients to self-monitor their treatment and status and provide information to healthcare managers which, in cooperation with health care providers, establish recommendations for treatment to optimize patient care.

Yet another objective of the present invention is to provide effective patient education using both conventional web-based user interfaces and high bandwidth interactive video. Effective patient education can facilitate client self-management improving clinical and economic outcomes. Interactive video can provide these services in an asynchronous mode with substantial cost savings. That is, clients can interact with the same video session many times without taking time from individual providers. If a person to person intervention is required, the individual provider can choose the appropriate time with the assistance of resource management tools provided by the disease management network rather than having to respond immediately to a large number of urgent and disruptive, but low priority queries.

These and other objects of the present invention are substantially achieved by providing a health outcomes and disease management network for efficiently and effectively monitoring patient status as well as providing recommendations for improved patient healthcare. The network can be configured, for example, as an Internet-based network which enables information to be efficiently exchanged between healthcare managers, healthcare providers, and patients. The network includes a centralized network comprising, for example, a computer or computer network, and a centralized database for storing information pertaining to the healthcare management network, such as patient information, treatment plan information, recommendations from care providers, recommendations from healthcare managers, and so on.

The centralized network communicates via the Internet, for example, to workstations that can be used by care managers, and workstations that can be used by care providers, such as physicians and specialized healthcare providers, so that the managers and providers can readily add and retrieve information to and from the database. The centralized network also communicates via the Internet, for example, to one or more IDNs or other payors. The network further includes terminals, such as interactive televisions or workstations, which can be provided to each patient subscribing to the network, and which communicate with the centralized network via the Internet, for example. The patients also can be provided with health monitoring tools, such as testing equipment and the like, which enable the patients to add and retrieve information to and from the centralized database via the patient workstation. When a patient is selected for participation in the network, information relevant to the patient's healthcare treatment as well as the patient's disease, if any, is collected and entered into the centralized database. A patient periodically updates this information as instructed by a healthcare manager who monitors the information provided by patients to which he or she is assigned. The healthcare manager is any individual provider that serves as the primary contact between a patient and the care team. The healthcare manager may be a staff person, a nurse, a physician assistant, or a medical doctor. Most preferably, the healthcare manager will be part of the local provider organization. The healthcare manager uses this information to coordinate treatment plans for the patient with the healthcare providers, and to provide suggestions and recommendations to the patient for improved overall healthcare. The healthcare manager further uses the information collected from the patients and providers to identify improvements in care that can be provided by adjusting the treatment plans for certain individual patients or groups of patients.

The above objects are also substantially achieved by providing a system for monitoring health-related conditions of patients, employing a plurality of remote monitoring stations and a computer network. Each of the remote monitoring stations includes at least one measuring device, adapted to measure a physiological condition of a respective patient, and to provide data representative of the physiological condition for inclusion among patient health-related data pertaining to a respective patient. The health-related data can include, for example, data relating to a patient's heart. The computer network comprises a database containing accumulated health-related data pertaining to health-related conditions and treatment. The computer network is adapted to receive the patient health-related data from the remote monitoring stations via, for example, the Internet, to establish treatments programs for the patients based on their respective patient health-related data and the accumulated health-related data, and to revise the accumulated health-related data based on the patient health-related data. The computer network further includes at least one data access device, adapted to provide a health care provider access to the computer network and the database. The computer network is adapted to generate reports, each including health-related information pertaining to a respective said patient. The computer network is adapted to provide the accumulated health-related data stored in the database to organizations financing at least a portion of the treatment programs, and is adapted to receive financial data pertaining to the treatment programs from the organizations and to store the financial data in the database.

### BRIEF DESCRIPTION OF THE DRAWINGS

These and other objects, advantages and novel features of the invention will be more readily appreciated from the following detail description when read in conjunction with the accompanying drawings, in which:

Fig. 1 is a conceptual block diagram illustrating a health outcomes and disease management network according to an embodiment of the present invention;

Fig. 2 is a conceptual business model diagram illustrating an example of the manner in which benefits flow between patients, healthcare providers, healthcare managers, payors and the network shown in Fig. 1;

Fig. 3 is flowchart illustrating general operations performed by the network shown in Fig. 1;

Fig. 4 is a flowchart illustrating specific operations performed by the network shown in Fig. 1;

Figs. 5-9 illustrate tables which provide descriptive information pertaining to the flowchart shown in Fig. 4;

Fig. 10 is a flowchart illustrating an example of general activities performed by a healthcare manager in the network shown in Fig. 1;

Fig. 11 is a flowchart illustrating an example of specific activities performed by a healthcare manager in the network shown in Fig. 1;

Fig. 12 is a flowchart illustrating an example of general activities performed by a client in the network shown in Fig. 1; and

Fig. 13 is a flowchart illustrating an example of specific activities performed by a client in the network shown in Fig. 1.

### DETAILED DESCRIPTION OF THE PREFERRED EMBODIMENTS

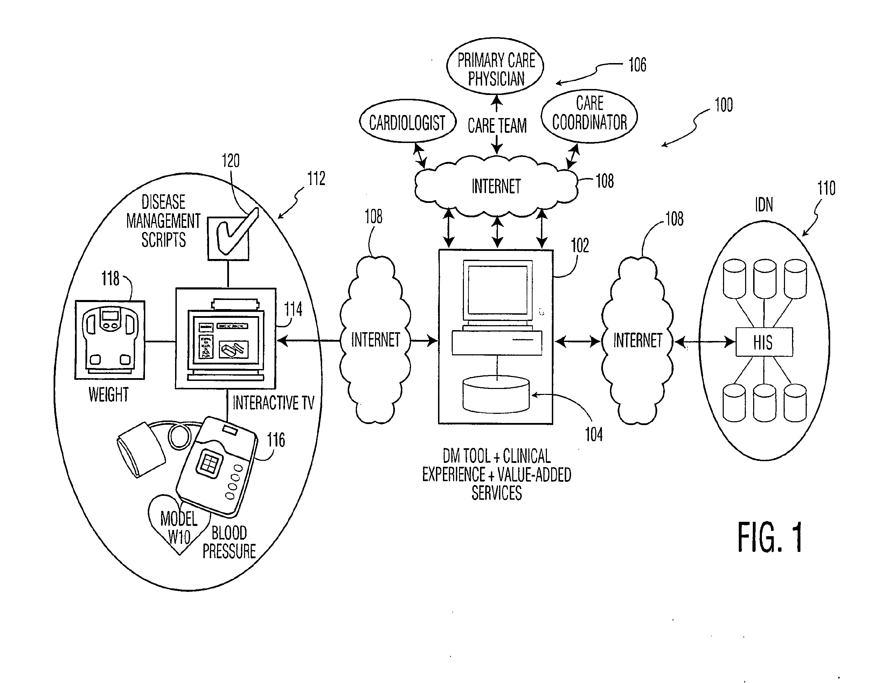

A health outcomes and disease management network 100 according to an embodiment of the present invention is shown in Fig. 1. As illustrated, the network 100 includes a centralized computer or computer network 102 including a centralized database 104 for storing information pertaining to patients in the network, as well as healthcare and health outcomes information. The network 100 is preferably an Internet-based network which enables parties participating in the network 100 to access the centralized database 104 via the internet. In this example, the network 100 is used to monitor heath outcomes and manage patients suffering from heart disease. However, the basic principles of the network 100 can be used for any health outcomes and disease management and patient education program.

The network 100 further includes one or more care teams 106 that include, for example, primary care teams (PCT), extended care teams (ECT) and an appropriate number of healthcare managers, the responsibilities of each are described in more detail below. For simplicity, only one care team 106 is shown. However, network 100 can include any suitable number of care teams, and each care team can include any suitable number of managers, a primary care team and extended care teams. The members of the care team 106 have access to workstations, such as personal computers, handheld devices, pagers, wireless phones or the like, which can interact with the centralized network 102 via the Internet 108 to access data in the centralized database 104 and to provide data to the centralized database 104 as described in more detail below. The centralized network 102 also communicates via the Internet with networks and databases 110 of one or more IDNs or other payors for purposes discussed in more detail below.

As further illustrated in Fig. 1, the network 100 further includes patients or clients 112 which receive treatment by the care team 106, and whose health status and outcomes are monitored by members of the care team 106 as described in more detail below. For simplicity, only one client 112 is shown in Fig. 1. However, as can be appreciated by one skilled in the art, the network can have any suitable number of clients, such as several hundred thousand or even millions, as long as the clients can be properly serviced by the care teams 106.

As further illustrated, the clients 112 each have access to a workstation 114, such as an interactive TV computer workstation or the like, which can access the centralized network 102 and centralized database 104 via the Internet 108. The workstation 114 serves the client 112 by providing automated answers to many questions about management of the client's disease state, as described in more detail below. The workstation 114 also enables such information to be communicated from the client 112 to centralized database 104 which provide services to a care team in charge of monitoring the client 112. Patient education may be "prescribed" through the healthcare network by a member of the care team or obtained through patient self-exploration using a home information appliance (interactive television, pager, wireless phone, handheld device).

The clients 112 are also each provided with monitoring or diagnostic tools, such as blood pressure measuring devices 116, electronic scales 118, and disease management information charts 120, as well as glucometers, thermometers, spirometers, medication management and various other devices which can be used to obtain diagnostic and assessment information from the clients 112. As described in more detail below, the clients 112 use these tools to enter information about themselves and their condition into the centralized database 104 and then communicate personal information via their workstations 114. The client may manually enter the information into the workstation 114, or this information may be transparently transferred between the devices and the workstation 114 by wireless or cabled means. Members of their care team 106 can review that information and monitor the clients' status as well as provide treatment information and recommendations to the client 112.

Specifically, the centralized network 102 interprets the diagnostic and assessment information and presents it to the appropriate care team 106 in a manner that both improves the provider's productivity and limits liability of information overload. As discussed in more detail below, the centralized network 102 stratifies clients 112 into various prioritized risk related groups for action by the care team. An interactive dashboard on the workstation can be used that profiles the client population by selected risk factors which may include time since last contact, assessment and diagnostic information (for example, blood glucose, glycated hemoglobin, weight, blood pressure), abnormal medication usage, significant comorbidities, psychosocial data, and quality measures. Tools are also provided to assist providers as they respond to this patient information including resource management, scheduling, guidelines, protocols, and behavioral tools. The provider team is much more productive since many of the routine diagnostic and assessment functions have been automated. Client information is then archived into a second database for further analysis to benchmark performance and identify opportunities for improvement of care practices. The model uses a blend of devices and services to maximize value for all the stakeholders.

Whenever information is transmitted the disease management network will maintain appropriate security and confidentiality of patient information. For example, the Health Insurance Portability and Accountability Act of 1996 (Public Law 104-191), also known as HIPAA, was enacted as part of a broad Congressional attempt at healthcare reform. HIPPA requires the United States Department of Health and Human Services (HHS) to develop standards and requirements for maintenance and transmission of health information that identifies individual patients. HIPPA both standardizes the interchange of electronic patient data for certain administrative or financial transactions, and specifies standards to ensure confidentiality of electronic health information. Home wireless networks must also ensure reliability and confidentiality of personal health information. This may be achieved through unique identification of personal devices and through encryption. Potentially, individual patients and providers, and other items important to patient health may be identified to the disease management network with RF tags. Aggregation of population based information will also require standards to ensure confidentiality through anonymity and contractual means similar to those in place for obtaining the Medicare Provider Analysis and Review (MEDPAR) file of inpatient hospital and Skilled Nursing Facility (SNF) final action stay records from the Health Care Financing Administration.

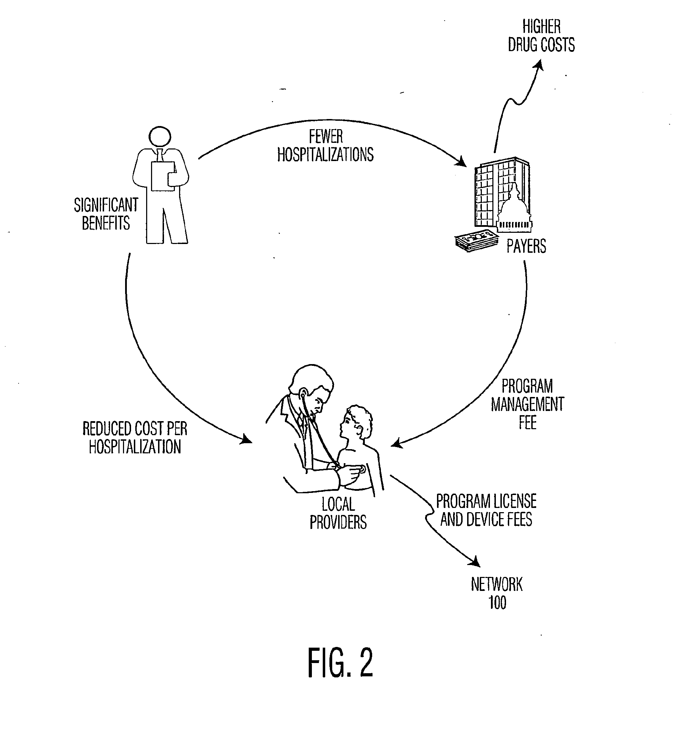

Fig. 2 is a conceptual business model diagram generally illustrating the benefits received by participants in the network 100. The business model functions as follows. Clients 112 receive services which confer significant wellness benefits. These services also confer financial benefits to payors who pay a management fee to local provider organizations. Payors may experience some increased cost due to improved utilization of drug therapies. The providers may pass through some of this fee to the network 100. The network 100 provides disease management protocols, frameworks, and technology to support local providers, including support to participate in clinincal trials. Local providers may charge a per patient per month (PPPM) fee, some of which may be passed on to the network 100 in return for on-going support. The network 100 also provides behavioral tools which assist providers with understanding and motivating patients.

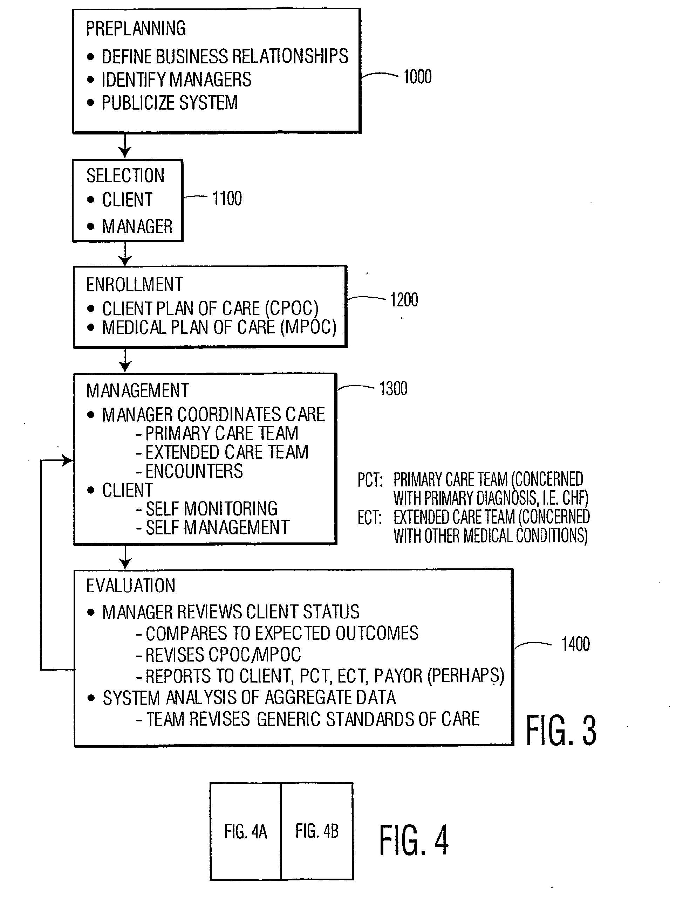

Fig. 3 is a flowchart providing a general illustration of activities performed by the network 100 and its participants. As shown in step 1000, the business relationships are defined that are to exist between care team members 106 (including medical doctors, nurse practitioners, nurses, and physical assistants), payors 110 and clients 112. The healthcare managers and their associated responsibilities will also be identified, and publicizing and marketing of the network 100 will be performed. The initial clients 112 are selected in step 1100 and assigned to respective health managers in the care team 106.

While the most preferred method of disease management is through a local provider and care team, we anticipate certain patients, their physicians, or their provider networks may independently elect to participate in aspects of the disease management network while administering other aspects independently. For example, the physician may elect to participate in a monitoring service using devices only while providing disease management services in her own practice. Likewise, certain clients may elect to participate in the disease management independently of their payors. These clients would self-pay these expenses.

In step 1200, the healthcare managers develop a respective client plan of care (CPOC) and medical plan of care (MPOC) for their respective clients 112 in cooperation with the care providers, such as the primary care physicians, hospitals and specialists. In step 1300, the healthcare managers accordingly care for their respective clients 112 between the primary care team and extended care teams, while also receiving, monitoring and evaluating information provided by the clients 112. During this time, the clients 112 are also responsible for monitoring and managing their conditions, and providing data to the centralized database 104. In step 1400, the healthcare managers review the status of their respective clients 112 and compare their client's progress to expected outcomes. The managers can coordinate with the care providers to revise the CPOCs and MPOCs for their respective clients 112, and report relevant information to the clients, PCTs, ECTs and payors, as necessary. The members of the care team 106 can then analyze the data received from all the clients 112 or a sample of clients, as appropriate, and revise the standards of care for particular health conditions, as deemed necessary.

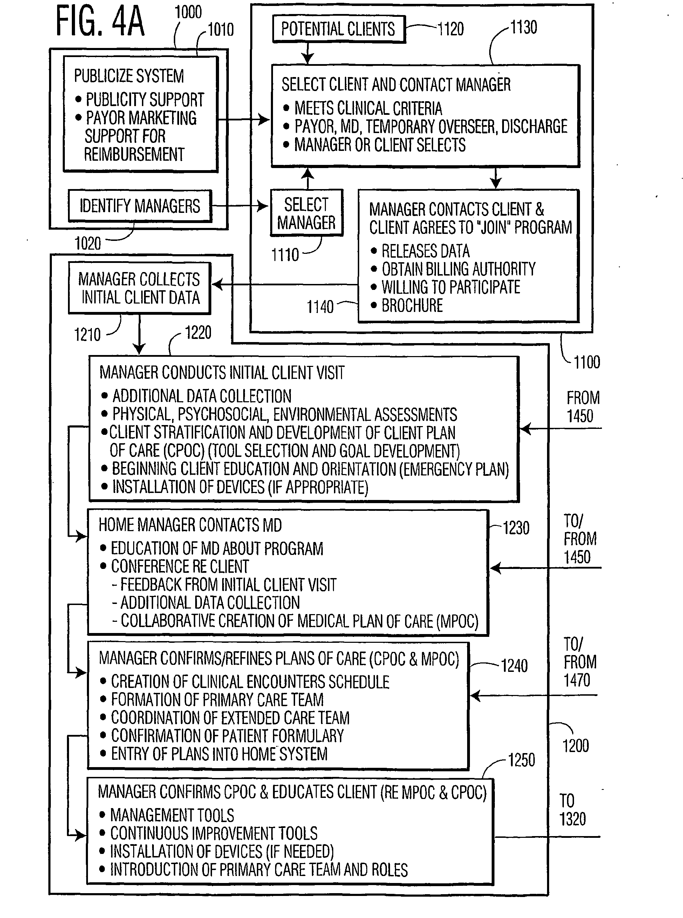

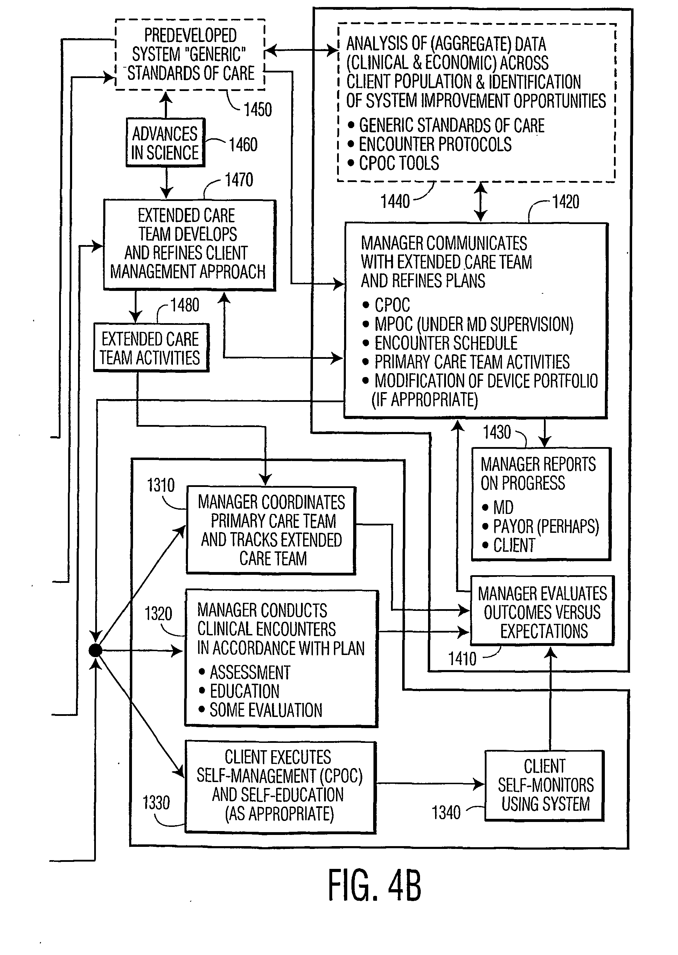

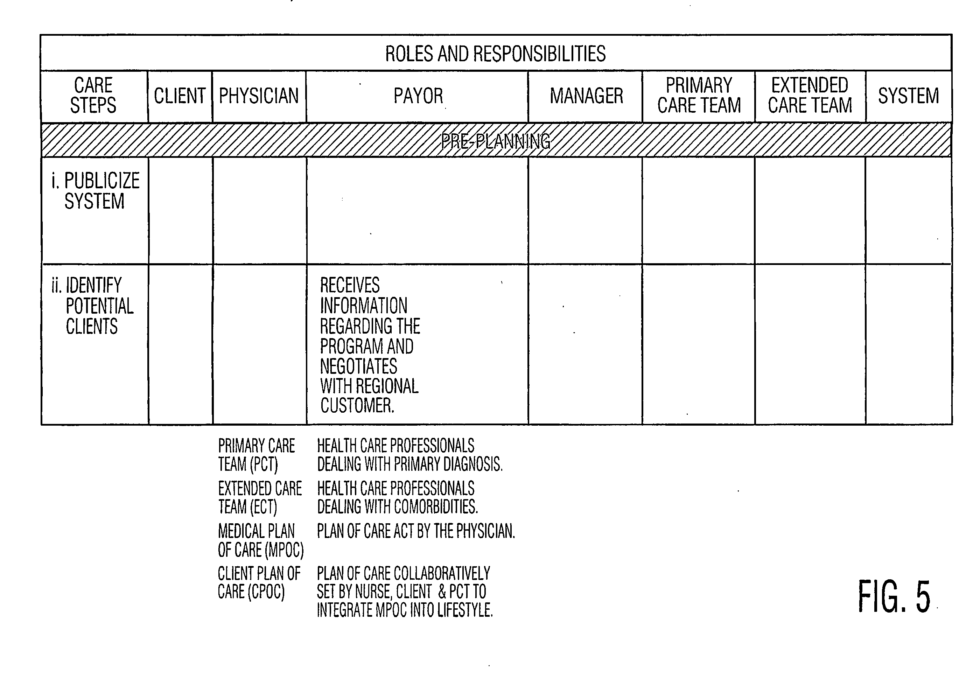

Fig. 4 illustrates a detailed flowchart of an example of activities performed during the general steps shown in Fig. 3, and the tables shown in Figs. 5-9 provide descriptive information pertaining to these activities. For instance, during the preplanning step 1000, the network 100 performs step 1010, which involves publicizing the network 100. In step 1020, healthcare managers are identified. Most preferably, the healthcare manager can be selected from the local provider organization.

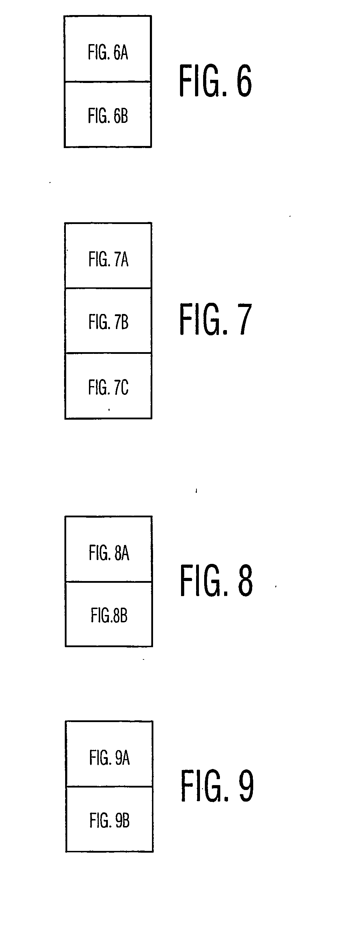

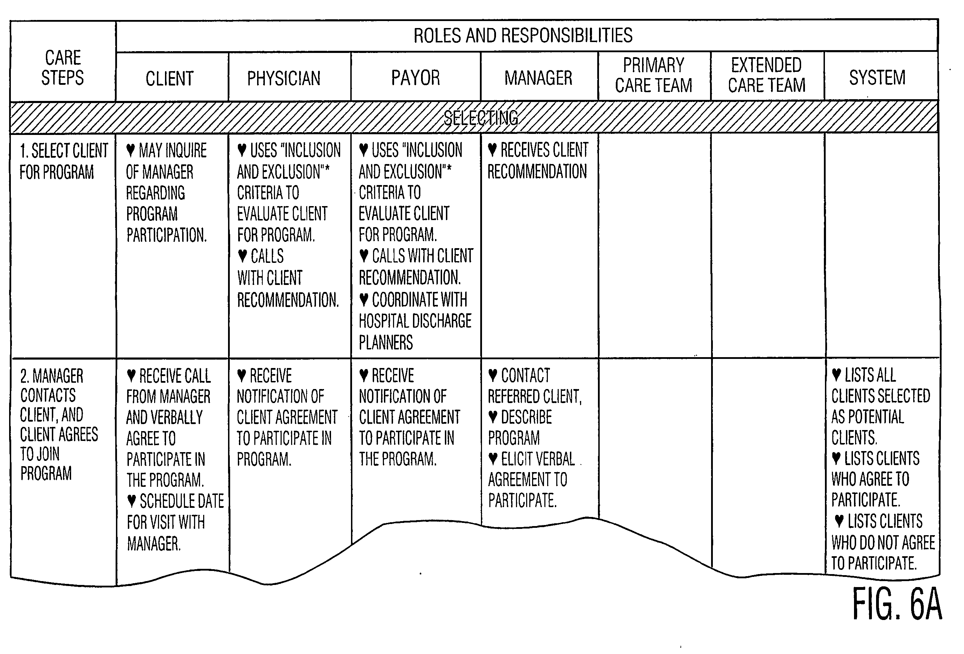

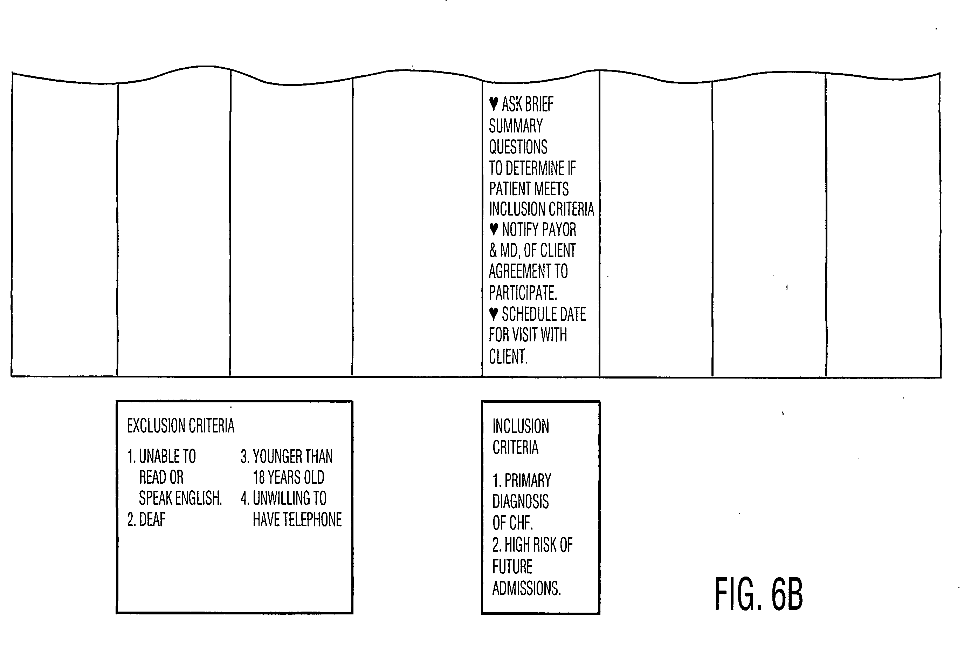

During the selection step 1100 shown in the flowchart in Fig. 3, several substeps shown in the flowchart of Fig. 4 and as described in the table in Fig. 6 are performed. For example, the health care managers are selected in step 1110 who are to manage certain groups of clients 112. In step 120, potential clients 112 are recognized for participation in the network 100, and in step 1130, the clients 112 are selected for participation in the network 100.

The client selection process is an interactive process between the client 112, healthcare manager, care providers and payors. For example, a client 112 wishing to participate in the network may make an inquiry to a healthcare manager regarding the details associated with participating in the network 100. Most preferably, a provider (hospital discharge manager, physician) will recommend a client for participation in the disease management network. A healthcare provider, such as a physician, uses inclusion and exclusion criteria to evaluate the suitability of a client 112 for participation in the program. For example, inclusion criteria for a patient participating in a heart disease monitoring program may have a requirement that the client 112 receive a primary diagnosis of congestive heart failure (CHF), and that the client 112 is at a high risk of future admissions to, for example, a hospital or a long-term healthcare facility. Exclusion criteria may include factors such as whether the potential client 112 is unable to read or speak English, is deaf, is younger than 18 years old, or is unwilling to have a telephone. The disease management network may ultimately incorporate features to overcome these limitations including multilingual capability, speech recognition and synthesis, wireless communications, and so on.

When a healthcare provider has analyzed a particular client 112 based on the inclusion and exclusion criteria, the healthcare provider can contact the healthcare manager with a recommendation to either accept or reject the client's participation. The payor can also use the same or similar inclusion or exclusion criteria to evaluate the suitability of a client 112 for participation in the program. Once an evaluation has been made, the payor can contact the healthcare manager with a recommendation to either accept or reject the client's participation in the program. The payor would also coordinate with, for example, a hospital discharge planer which will oversee the release of client 112 from the hospital or long-term care facility. In all cases, the intent will be to maintain control of the client's primary care physician or cardiologist. The disease management network will enhance and extend the reach of the existing medical decision markers.

Once the information has been gathered and the recommendations have been made in step 1130, the healthcare manager contacts the client 112 in step 1140, and the client 112 can then agree to participate in the network 100. For example, the client 112 can receive a call or an e-mail from the healthcare manager and agree to participate in the network verbally and through written signed consent, via return e-mail and/or regular mail. The client 112 can then schedule a date for a visit with the healthcare manager.

When the client 112 has agreed to participate in the network 100, the healthcare provider, such as a physician, receives notification from the network 100 of the client's agreement to participate. The payor 110 also receives notification of the clients' participation. Furthermore, while the client 112 is in communication with the healthcare manager, the healthcare manager can describe the program to the client 112 and solicit questions from the client 112 to determine if the client 112 actually meets the criteria for participation. If the healthcare manager agrees after receiving this additional information that the client 112 is suited for participation, the healthcare manager coordinates with the client 112 to schedule a date for a visit with the client 112.

Also during this process, the information being provided by the client's, healthcare providers, payors and healthcare managers is being collected at the central network 102 and in the central database 104. The central network 102 can then generate lists of clients 112 that are selected as potential clients, as well as groups of clients 112 who have agreed to participate in the program and groups of clients 112 who have not agreed to participate in the program. The members of the care team 106 and payors 110 can readily access this information via the Internet using their workstations.

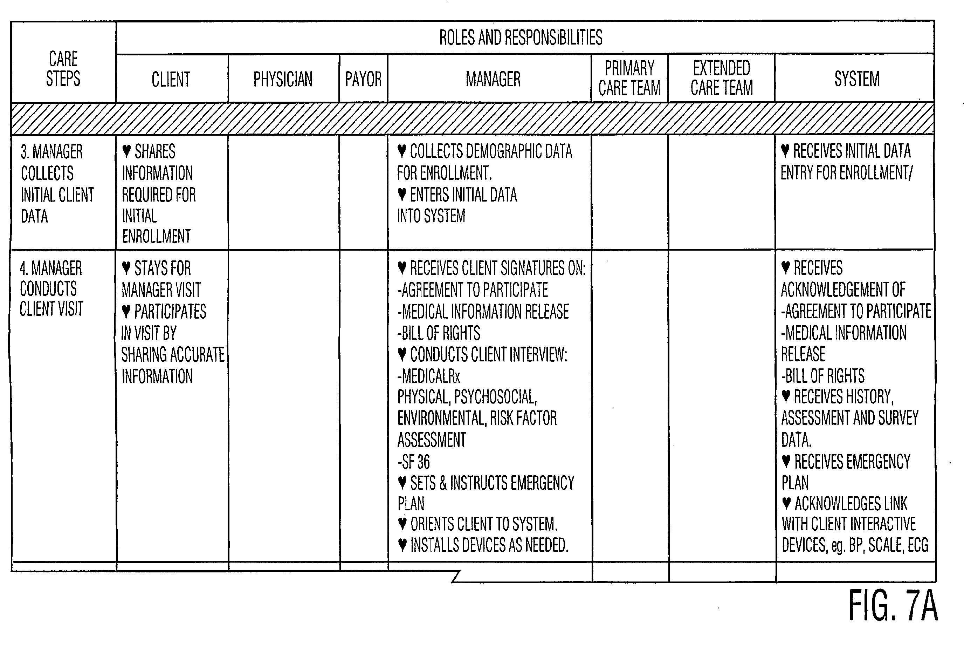

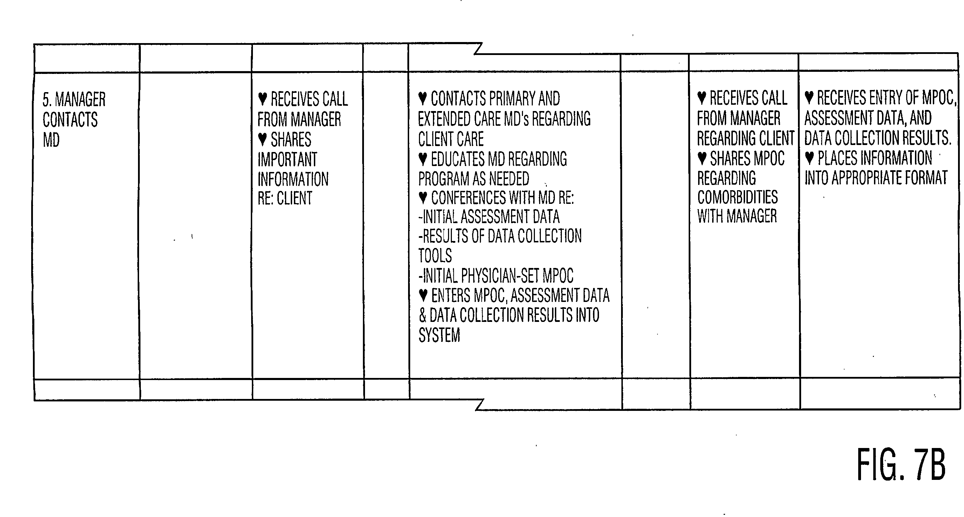

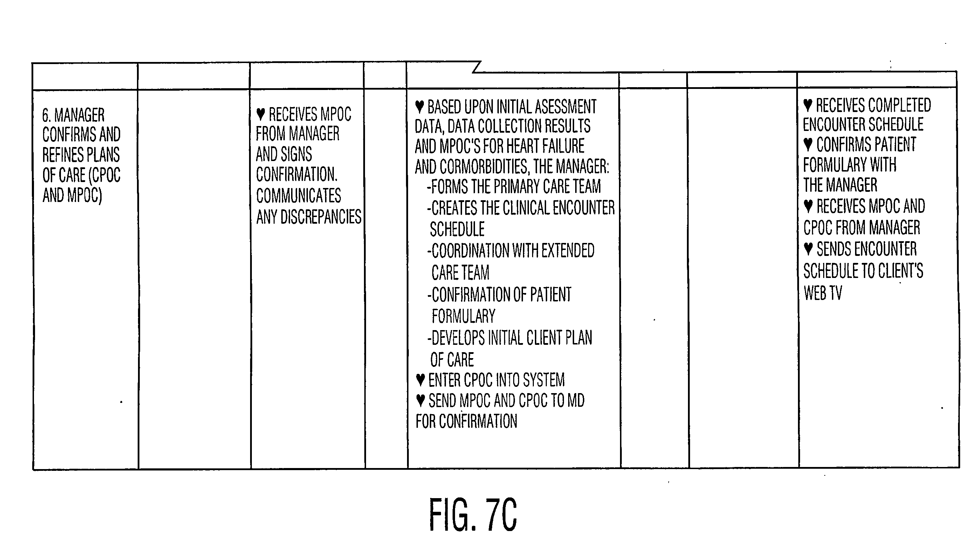

Once the substeps of step 1100 have been performed, the process proceeds to the enrollment phase in step 1200 shown in Fig. 3, and the following substeps are performed as shown in Fig. 4 and as described in the table in Fig. 7. Specifically, in step 1210, the manager collects the initial data from the client 112. For example, the client 112 will provide the manager with information required for initial enrollment. The healthcare manager collects demographic data for the enrollment and enters this initial data into the database 104, which receives and categorizes this data as appropriate. During this step, support can be provided to the healthcare manager concerning system utilization and troubleshooting.

In step 1220, the healthcare manager may conduct a visit with the client 112. The client 112 can stay at home while the healthcare manager visits so that the client 112 can share the appropriate information with the healthcare manager in a comfortable environment. Alternately, the interview may be conducted from a remote location using any normal means of telecommunication (for example telephone, e-mail, wireless phone). The healthcare manager also conducts a client interview which includes an evaluation of the client's medical condition as well as the client's physical and psychosocial conditions, and environmental conditions.

During the client visit, the healthcare manager can also perform a risk factor assessment. The healthcare manager then can set an emergency treatment plan for the client 112 and provide the client 112 with the necessary instructions to implement such a plan. The healthcare manager also orients the client 112 to the network, and may install any of the devices shown in Fig. 1, such as a blood pressure monitoring kit 116, an electronic scale 118, interactive TV equipment and the like. Alternately, less acute patients may be monitored through clinic visits on an outpatient basis. Support can be provided to the healthcare manager in setting up the client's equipment and for troubleshooting malfunctions in the equipment. The centralized network 102 of the network receives acknowledgement of the client's agreement to participate, the client's medical release and the client's acknowledgment of the client's bill of rights. The database receives the data provided by the client 112, and the centralized network 102 receives the emergency treatment plan. The centralized network 102 can then acknowledge the link via, for example, the internet 102, with the client 112 interactive devices, such as the blood pressure monitoring kit 116, electronic scale 118, and the like.

In step 1230, the healthcare manager contacts the healthcare provider, such as the clients' primary care physician. The physician receives a telephone call, for example, from the healthcare manager, and then the healthcare manager and physician share important information pertaining to client 112. The healthcare manager also can contact the extended care physician regarding the client's healthcare and can confer with the physicians concerning the initial assessment data, the results of the data collection from the client 112, and the initial MPOC set by the physician. The healthcare manager can also educate the physician as necessary with regard to the attributes of the network 100.

The manager also enters the MPOC, assessment data and data collection results into the centralized network 102 and centralized database 104 of the network 100. The centralized network 102 upon receiving this information places the information in the appropriate format. The extended care team also share the MPOC regarding comorbidities with the healthcare manager. Support can be provided for the health care manager regarding utilization and troubleshooting.

The process continues to step 1240 where the healthcare manager confirms and redefines plans of care for the client 112, such as the CPOC and MPOC. The physician receives the MPOC from the healthcare manager and can confirm the MPOC or communicate any discrepancies in the MPOC with the healthcare manager. The healthcare manager forms the primary care team based on the initial assessment data, data collection results and MPOC and comorbidities received in step 1230. In doing so, the healthcare manager creates a clinical and encounter schedule, develops an initial client plan of care, establishes coordination of the care with the extended care team, and confirms the patient formally.

The health care manager enters the CPOC into the centralized network 102 and database 104 of the network, and as discussed above, sends the MPOC and CPOC to the physician for information. The centralized network 102 and database 104 receives the MPOC and CPOC from the healthcare manager, and the centralized network 102 confirms the patient's familiarity with the healthcare manager. The centralized network 102 then sends the encounter schedule to the client's workstation 114 via the Internet 108 so that the client 112 can view the encounter schedule at his or her workstation 114, which can be a computer terminal or interactive TV as described above. At all times, support can be provided for the health care manager for system utilization and troubleshooting purposes.

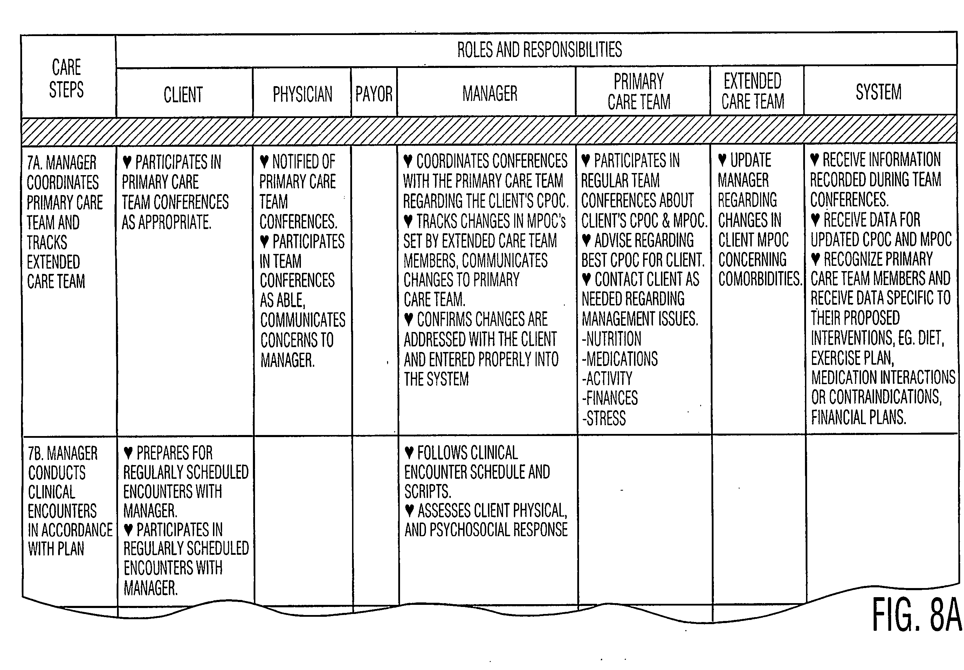

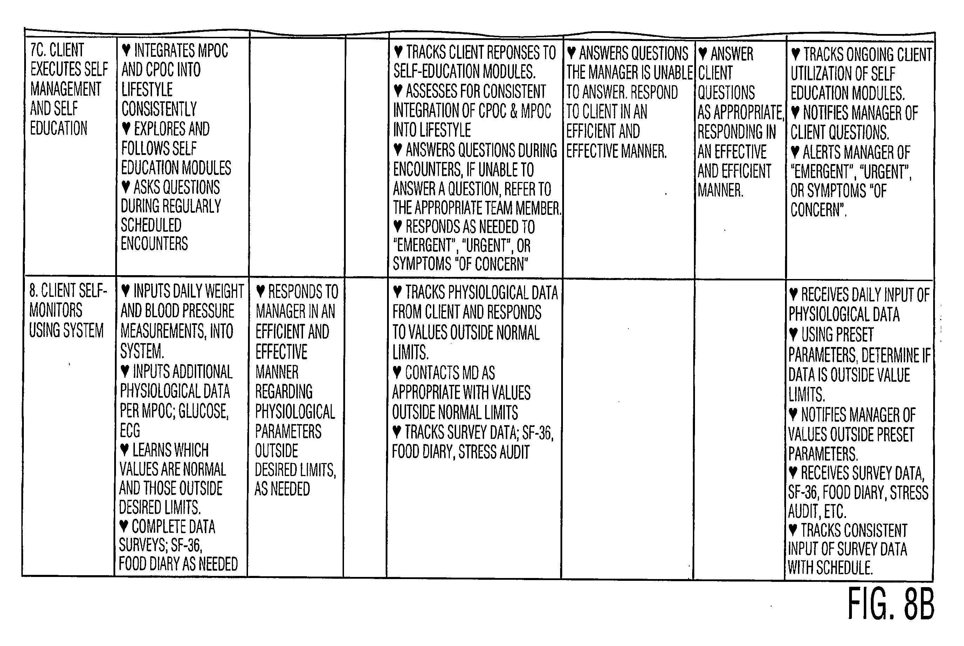

Once the processes in the enrollment 1200 have been performed as described above, the process proceeds to the management steps 1300 shown in the flowchart of Fig. 3. The substeps of the manager process 1300 are then performed in the following manner as shown in flowchart of Fig. 4 and as described in the table shown in Fig. 8.

For example, steps 1310, 1320 and 1330 can be performed simultaneously by the different parties involved. That is, in step 1310, the healthcare manager coordinates the primary care team and the extended care team. During this time, the healthcare manager coordinates conferences with the primary care team regarding the client's CPOC, tracks changes in the client's MPOC set by the extended care team members, and communicates the changes to the primary care team. The healthcare manager also confirms that changes are address with the client 112 and entered properly into the centralized network 102.

Also during this time, the client 112 participates in primary care team conferences as appropriate, while the physician is notified of primary care team conferences and participates in them as appropriate. The physician also can communicate concerns regarding the client's condition or health management to the healthcare manager. The primary care team participates in a regular team conferences about the client's CPOC and MPOC, and provides advice regarding the best CPOC for the client 112.

The primary care team can also contact the client 112 as necessary regarding management issues, such as nutrition, medications, physical activity, finances, stress, and the like. The extended care team updates the healthcare manager concerning changes in the client's MPOC concerning comorbidities. The centralized network 102 receives the information recorded during the team conferences, as well as data for updated CPOC and MPOC. The centralized network 102 recognizes primary care team members and receives data specific to their proposed interventions, such as changes in the client's diet, exercise plan, medication interactions or contra indications, and financial planning. During this time, customer service support can be provided to the healthcare manager regarding system utilization and troubleshooting.

In step 1320, the healthcare manager conducts the clinical encounters in accordance with the healthcare plan. Specifically, the healthcare manager follows clinical encounters schedules and scripts and assesses client's physical and psychological responses. The client 112, on the other hand, prepares for regular encounters with the healthcare managers and participates in those regularly scheduled encounters. Customer service support can also be provided to the healthcare manager as necessary.

In step 1330, the client 112 executes self-management and self-education. That is, the client 112 integrates the MPOC and CPOC into his or her lifestyle consistently, while exploring and following self-education modules and asking questions during the regularly scheduled encounters with the healthcare manager. The healthcare manager tracks client's responses to self-education modules, assesses the responses for consistent integration of CPOC and MPOC into the client's lifestyle, and answers questions during the encounters. If the healthcare manager is unable to answer a question, the healthcare manager can refer the questions to the appropriate team member. The healthcare manager can also respond as necessary to emergency or urgent conditions, or symptoms of concern.

During this time, the primary care team can answer client questions that the manager is unable to answer, while responding to the client 112 in a efficient and effective manner. The extended care team can also answer questions as appropriate, and respond in an effective and efficient manner. In this way, the primary team consists of any person concerned with the client's primary diagnosis. The primary care team may include the client, the client's general practitioner, primary care physician, or cardiologist, nurse practitioners, pharmacists, dieticians, social workers and others. The extended care team is concerned with secondary comorbidities. For example, if the primary diagnosis is heart failure, a member of the extended care team might include an endocrinologist treating diabetes, a comorbidity.

While these activities are being performed, the centralized network 102 tracks ongoing client utilization of self-education modules, notifies the healthcare manager of client questions, and alerts the healthcare manager of emergency, urgent, or symptoms of concern. Customer service can also be provided during this time as appropriate.

In step 1340, the client 112 performs the self-monitoring of his or her condition using the equipment provided such as the blood pressure measurement equipment 116, electronic scale 118, and the like. For instance, the client 112 can enter daily weight and blood pressure measurements, which can be forwarded to the centralized network 102 via the client's workstation 114, and that data can be entered into the centralized database 104. The client 112 can input additional physiological data through the MPOC, such as glucose measurements, ECG, and so on. The client 112 learns which values are normal and which are outside desired limits, and completes data surveys, such as an SF-36, and a food diary, if appropriate. The client 112 provides all this information to the centralized network 102 via the client workstation 114.

During this activity, the healthcare manager tracks physiological data from the client 112 and responds to values outside the normal limits. The healthcare manager can contact the physician as appropriate with values outside the normal limits, and tracks the survey data provided by the client 112 in the SF-36, food diary, or a stress audit. The physician can respond the healthcare manager in a efficient and effective manner regarding physiological parameters outside the desire limits, as appropriate. During this time, the centralized network 102 is receiving input of the data and using preset parameters to determine if the data is outside normal limits. The centralized network 102 notifies the healthcare manager of the data that is outside the limits, and receives, stores and tracks the data provided by the client 112. At all times, customer services and support can be provided as necessary.

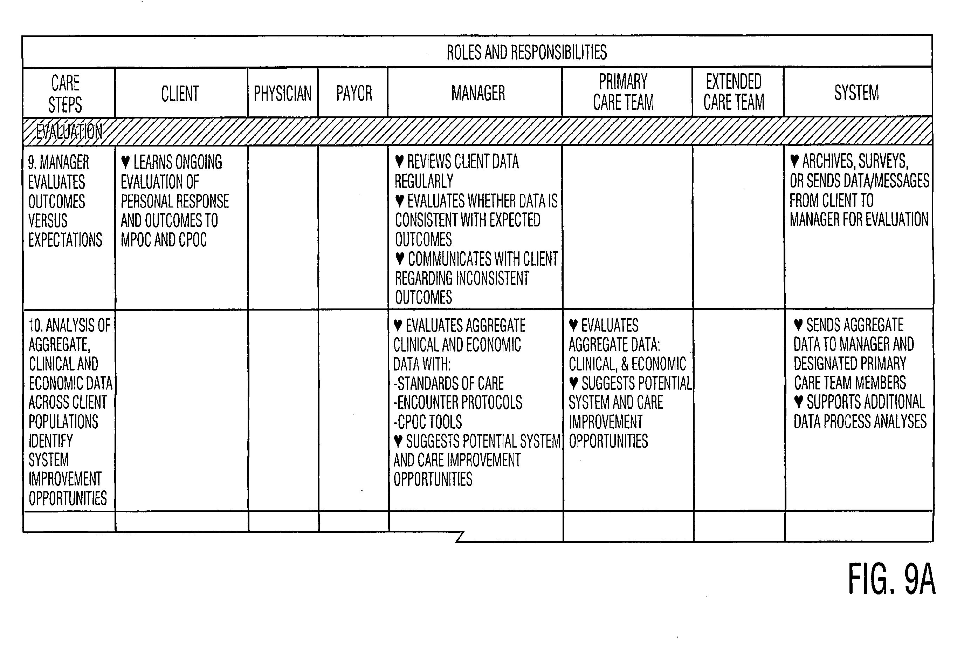

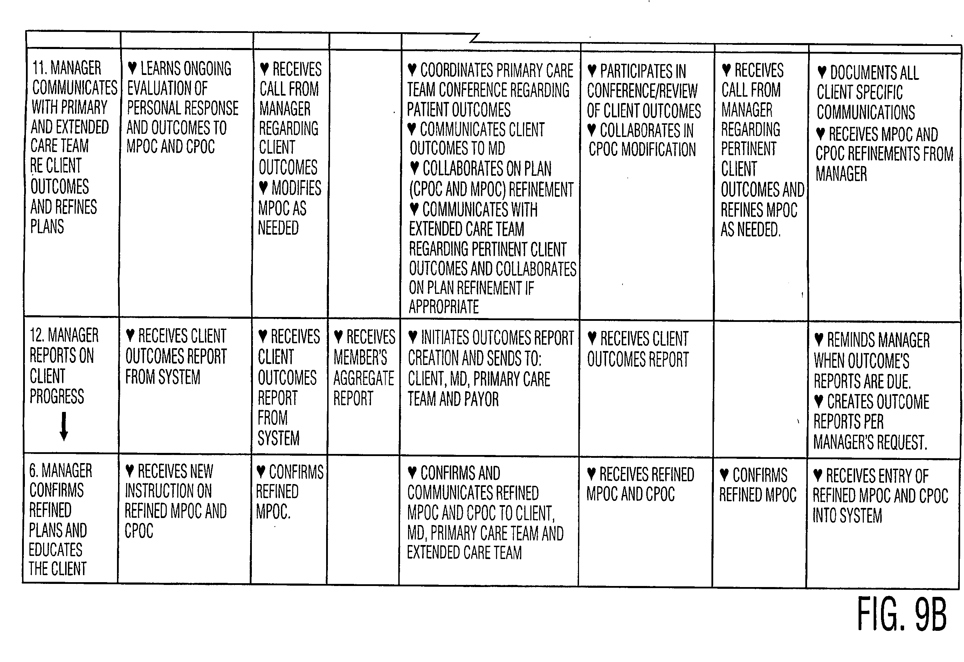

As further shown in Fig. 4, the evaluation process 1400 shown in Fig. 3 includes several sub processes as described in the flowchart in Fig. 9. For example, in step 1410, the healthcare manager evaluates outcomes of the client's progress verses client expectations. Specifically, the healthcare manager reviews the data from the client 112 regularly, evaluates whether the data is consistent with expected outcomes, and communicates with the client 112 regarding inconsistent outcomes. The data is archived and surveyed by the centralized network 102, which can send data messages from the client 112 to the healthcare manager for evaluation. The client 112 also learns of ongoing evaluation and receives personal responses from the healthcare manager, as well as outcomes relating to the MPOC and CPOC. Customer support can be provided during this process as necessary.

In step 1420, the healthcare manager communicates with the primary and extended care teams regarding client outcome and can, if necessary, define the treatment plans. For instance, the healthcare manager coordinates a primary care team conference regarding the patient outcomes, and communicates client outcomes to the physician. The physician can collaborate with the healthcare manager on defining the CPOC and MPOC for the client 112, and can communicate with the extended care team regarding pertinent client outcomes. The extended care team receives a contact from the healthcare manager regarding the pertinent client outcomes and can collaborate with the healthcare manager and, if necessary, the primary care team to define the MPOC if necessary. During this time, the centralized network 102 is receiving and documenting all client-related communications, as well as MPOC and CPOC refinements from the healthcare manager. Customer service support can also be provided. At this time, the client 112 also learns of ongoing evaluation of his or her personal responses, and outcomes to his or her MPOC and CPOC.

In step 1430, the healthcare manager reports on the client's progress. For example, the healthcare manager can interact with the centralized network 102 to create an outcomes report, and to send the report to the client 112, physician, primary care team and payor, for example. The primary care team, physician and client 112 receive the report from the healthcare manager. The centralized network 102 can remind the healthcare manager when the outcome reports are due. At this time, customer service can be provided to the healthcare manager, as well as administration, marketing, research and development. Updated data can be received on all of the network's clients, and bench marking can be done for clinical and economic outcomes.

It is also noted that in step 1440, the healthcare manager can analyze the aggregate, clinical and economic data cross client populations and identify improvements that can be made in client healthcare. For instance, the healthcare manager can evaluate aggregate clinical and economic data with regard to standards of care, encounter protocols, CPOC tools, and so on, and suggest potential improvements that can be made to the network 100 and overall healthcare provided. The primary care team can evaluate the aggregate data, as well as the clinical and economic data and also suggest improvements that can be made.

The centralized network 102 provides the data necessary to perform these evaluations to the healthcare manager and the designated primary care team members and provide support for additional data process analysis. At this time, the aggregate report data can be reviewed for improvements. The advisory board can consider the improvement suggestions and provide a recommendation for a pertinent plan of action. If appropriate, the improvements are incorporated into the network as well as into the generic standards of care that have been predeveloped and are shown in the activities box labeled 1450. As indicated, these standards of care are influenced by advances in science shown in the box labeled 1460, which influences the client management approach developed by the extended care team and extended care team activities as indicated in boxes 1470 and 1480.

Although Figs. 3 and 4 show the steps in a particular order, certain steps can be performed simultaneously, and the steps can otherwise be performed in any practical order which will achieve the intended outcome.

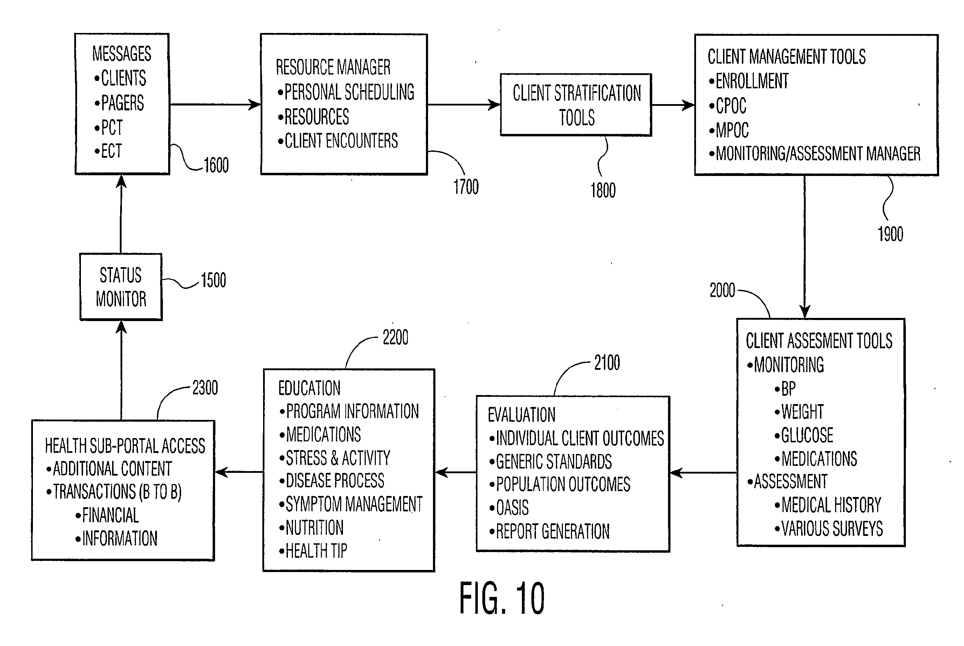

Fig. 10 is a flowchart illustrating activities and operations performed by a healthcare manager. Many of these steps are included in or compliment the steps shown in the flowchart of Fig. 4 described above. That is, as shown in step 1500, the healthcare manger will monitor the status of the clients 112 he or she is managing. In step 1600, the healthcare manager can receive messages from any of a number of sources, such as clients, pager messages, or messages from the PCT or ECT. If appropriate, the healthcare manager performs resource management activities in step 1700, which include personal scheduling of meetings with clients 112, management of resources and client encounters. In step 1800, the healthcare management can use client stratification tools to analyze the status of the clients being managed. In step 1900, the healthcare manager can use client management tools, such as those associated with enrollment, as well as the CPOC, MPOC and monitoring or assessment management tools.

In step 2000, the healthcare manager can use client assessment tools to assess the status of the client's condition. For example, the manager can use tools for monitoring the client's blood pressure, weight, glucose measurement and medications, and can use assessment tools which consider the client's medical history and results of various surveys relevant to the client's treatment. In step 2100, the healthcare manager performs evaluation activities, which include evaluation of individual client outcomes, evaluation of the generic healthcare standards used in the network 100 and outcomes of patients and the general population. The healthcare manager can also use an OASIS tool (Outcomes Assessment Information Set) and generate reports. Automated support for other regulatory, reporting, and billing requirements may also be provided including those required by the Joint Commission on Accreditation of Healthcare Organizations (JACHO), the National Committee for Quality Assurance (NCQA) / Health Plan Employer Data and Information Set (HEDIS), the Prospective Payment System (PPS), and Medicare/Medicaid. OASIS evaluation of each patient is required by the HCFA for reimbursement to home care agencies.

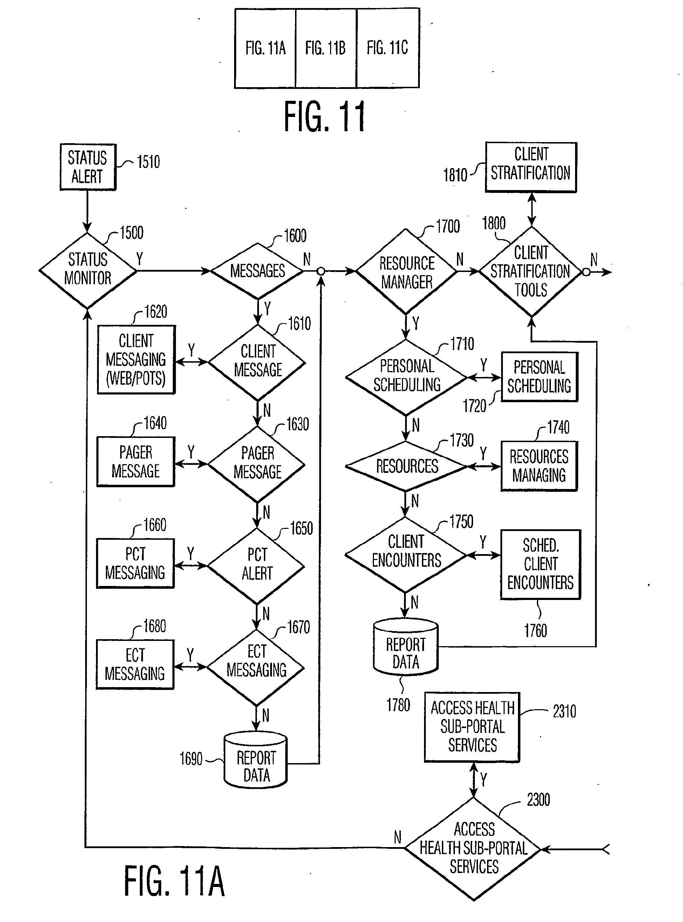

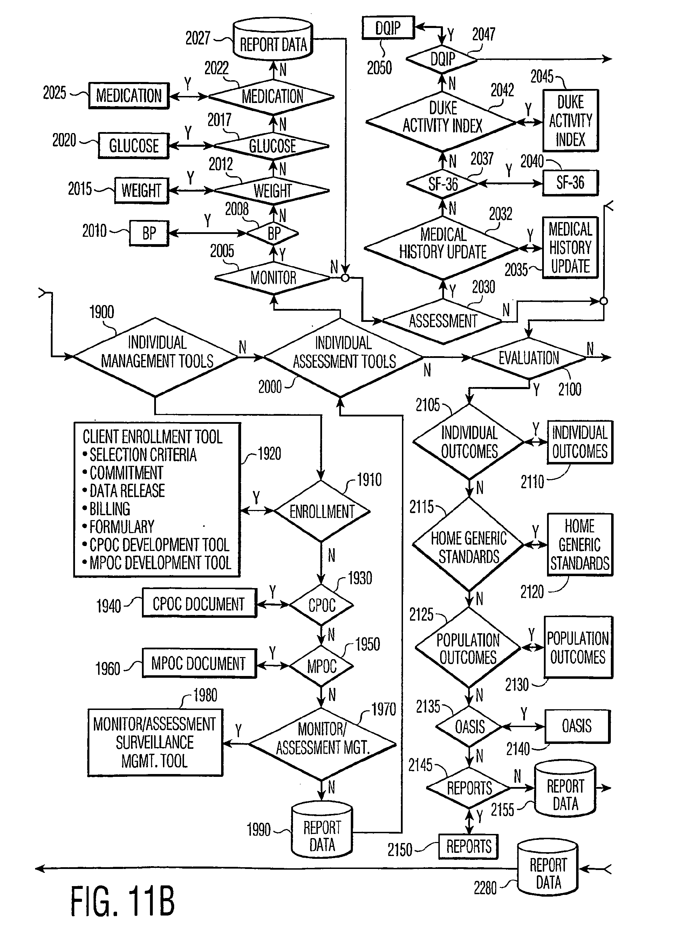

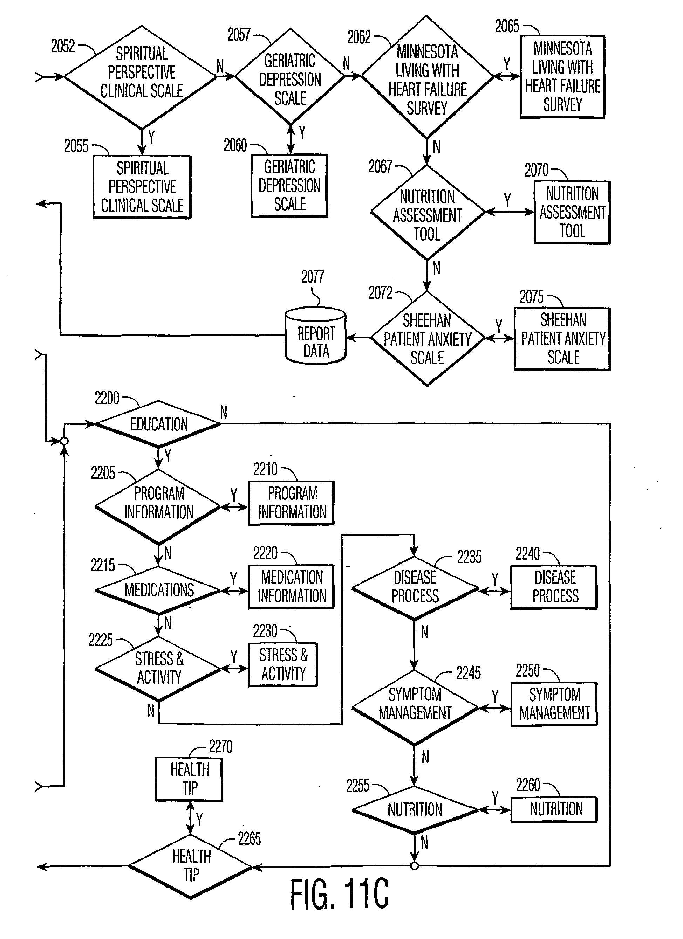

In step 2200, the healthcare manager can perform education-related activities, and in step 2300, the healthcare manager can perform healthcare sub-portal access activities. This operation will now be described in more detail with respect to Fig. 11.

In particular, during the status monitor step 1500, the healthcare manager waits for a status alert which comes, for example, from a client 112 or care provider. When a status alert has been received, the manager can then receive a message in step 1600. As illustrated, the healthcare manager determines in step 1610 whether the message was from a client 112. If so, the healthcare manager takes the appropriate action in step 1620 to handle client messages. If the message was not a client message, the healthcare manager determines if the message was received by his or her pager in step 1630. If so, the healthcare manager responds to the pager message as appropriate in step 1640. In similar manner, the healthcare manager determines in step 1650 if he or she has received an alert from the PCT. If so, the healthcare manager takes the appropriate action in step 1660.

The healthcare manager also determines whether the message has been received from the ECT in step 1670. If so, the healthcare manager takes the appropriate action in step 1680. It is noted that when the healthcare manager takes the appropriate action associated with a particular type of message in step 1620, 1640, 1660 and 1680, the healthcare manager can still check for additional types of messages. That is, the healthcare manager can receive client messages, pager messages, PCT alerts and ECT messages pertaining to a particular client, and can take the appropriate action for each message in steps 1620, 1640, 1660 and 1680. Once the healthcare manager has considered all of the messages and has performed the appropriate action, the healthcare manager reports the appropriate information to the centralized network 102 which is then stored in the centralized database 104.

In step 1700, the healthcare manager can take the appropriate resource management steps as described briefly above. That is, in step 1710, the healthcare manager determines whether any personal scheduling with, for example, clients, needs to be done. If so, the healthcare manager does the scheduling in step 1720. In step 1730 the healthcare manager determines whether any resources need to be allocated. If so, the healthcare manager performs the appropriate resource managing activities in step 1740.

In step 1750, the resource manager determines whether any client encounters need to be performed. If so, the resource manager schedules the client encounters in step 1760. As illustrated, once the healthcare manager has performed the activities in steps 1720, 1740, and 1760, as appropriate, the healthcare manager reports the data as necessary to the centralized network 102 in step 1780, and the centralized network can store that data in a appropriate format in the database 104.

The healthcare manager can than use the appropriate client stratification tools in step 1800 to perform the client's stratification operations in step 1810. The healthcare manager then can use the management tools in step 1900 to perform the appropriate managing activities, if necessary. For example, the healthcare manager can determine in step 1910 whether any enrollment operations need to be performed. If so, the healthcare manager can use the appropriate enrollment tools in step 1920. The healthcare manager can determine whether any CPOC has been established and if so, can obtain the CPOC document in step 1940. In step 1950, the healthcare manager can determine whether any MPOC has been established and, if so, can obtain the MPOC document in step 1960. In step 1970, the healthcare manager determines whether any monitoring or assessment management activities need to be performed. If so, such activities are preformed in step 1980. Once the above operations have been performed in steps 1920, 1940, 1960 and 1980, as appropriate, the healthcare manager can provide the appropriate data to the centralized network 102 in step 1990. The centralized network 102 can then store the data in centralized database 104, as necessary, and permit access to the data by the care team and payors. For example, payors can access the data to collect the data from all clients who are the payor's responsibility. The payors can then statistically review the collected data to determine, for example, whether any client's should receive reimbursements, and for outcomes analysis, preapproval of claims, and so on.

In step 2000, the healthcare manager uses the appropriate tools for monitoring and assessing the status of the clients 112. For example, if the healthcare manager determines in step 2005 that monitoring steps are to be performed, the manager will determine whether the client's blood pressure needs to be monitored in step 2008. If so, the manager will monitor the client's blood pressure reading in step 2010. The manager will also determine in steps 2012, 2017 and 2022 whether the client's weight, glucose measurement and medication, respectively, need to be monitored and will monitor the weight measurement, glucose measurement and medication in steps 2015, 2020 and 2025 respectively, as necessary. Once the appropriate monitoring activities have been performed, the manager reports the data in step 2027 to the centralized network 102, which can store the data in database 104.

The manager then determines in step 2030 whether any client assessment activity needs to be performed. If so, the manager determines in steps 2032, 2037, 2042 and 2047 whether any medical history updates, SF-36 forms, Duke Activity Indexes or DQUIPs, respectively, need to be used. Standard Form-36 (SF-36) available at www.sf36.com is an assessment tool used to determine quality of life and indicates both physical and psychosocial client status. SF-36 does not evaluate sleep adequacy, cognitive functioning, sexual functioning, health distress, family functioning, self-esteem, eating, recreation/hobbies, communication, and symptoms/problems that are specific to one condition. Symptoms and problems that are specific to a particular condition are not included in the SF-36 because the SF-36 is a generic measure. Consequently, SF-36 may be used to indicate quality of life in widely divergent populations and conditions, but is less useful for more targeted evaluations. The Duke Activity Index assesses the client's ability to perform routine physical activities. Other tools are targeted to specific disease states. For example, The Diabetes Quality Improvement Project (DQIP) represents a set of guidelines agreed upon by both the NCQA and the Foundation for Accountability (FACCT) as the standard for quality diabetes care. If any of these tools need to be used to assess the client's status, the manager obtains and uses the appropriate tools in steps 2035, 2040, 2045 and 2050, as necessary, and then continues to step 2052.

In steps 2052, 2057, 2062, 2067 and 2072, the manager determines whether any spiritual perspective clinical scales, geriatric depression scales, Minnesota Living with Heart Failure Surveys, nutrition assessment tools or Sheehan patient anxiety scales, respectively, need to be used. If any of these tools need to be used to assess the client's status, the manager obtains and uses the appropriate tools in steps 2055, 2060, 2065, 2070 and 2075, as necessary, and then continues to step 2077. In step 2077, the manager reports the data obtained from the use of all the tools described above to the centralized network 102, which stores the relevant data in the centralized data base 104.

The manager's activity then proceeds to the evaluation process 2100. During the evaluation process, the manager determines in steps 2105, 2115 and 2125 whether individual client outcomes, generic standards and population outcomes, respectively, need to be evaluated. If any of these outcomes require evaluation, the manager evaluates the outcomes as appropriate in steps 2110, 2120 and 2130, respectively.

The manager then determines in step 2135 whether an OASIS reimbursement process for payors should be performed. If so, the manager performs the OASIS reimbursement process in step 2140. In step 2145, the manager determines whether any reports on the above evaluations or process should be generated. OASIS evaluations constitute a significant portion of the documentation required under the Prospective Payment System (PPS). PPS is the proposed government reimbursement mechanism for home care agencies. If so, the manager generates the reports in step 2150. Once the necessary evaluations and processes have been performed and the appropriate reports, if any, have been generated, the manager reports the data to the centralized network 102 in step 2155, which stores the data in the centralized data base 104 as appropriate.

The manager's activity then proceeds to the education process in step 2200. During the evaluation process, the manager determines in steps 2205, 2215, 2225, 2235, 2245 and 2255 whether information relating to the client's treatment program, medication, stress and activity, disease process, symptom management and nutrition, respectively, should be conveyed to the client 112. If any of this information should be conveyed, the manager provides the information to the client 112 in steps 2210, 2220, 2230, 2240, 2250 and 2260, respectively, as appropriate. The manager then determines in step 2265 whether helpful heath tips should be provided to the client 112 in step 2270. The manager then reports the data obtained in the above steps to the centralized network 102, which can store the data in the centralized data base 104 as necessary.

The manager's activity then proceeds to the health sub-portal services process in step 2300. Health sub-portals provide links to various other information services including Medscape, WebMD, and Oncolink. If the manager determines that sub-portal services should be accessed, the services are accessed in step 2310. The manager then continues to monitor for additional status alerts in step 1500, and the process repeats as above for each status alert received.

Although Figs. 10 and 11 show the steps in a particular order, certain steps can be performed simultaneously, and the steps can otherwise be performed in any practical order which will achieve the intended outcome.

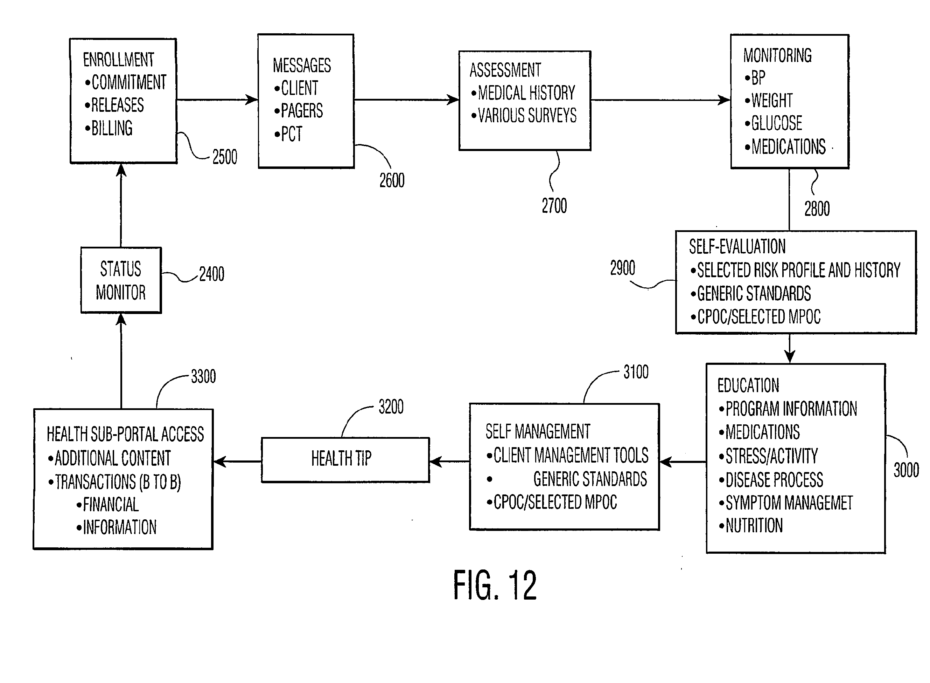

Fig. 12 is a flow chart illustrating general activities performed by a client 112 participating in the network 100 viewed from the perspective of the client 112. These steps are included in or complement the steps shown in the flowchart of Fig. 4 as described above. For example, the client 112 monitors for a status alert in step 2400. When the client 112 receives a status alert from the network, the client 112 can perform the appropriate enrollment activity, when applicable, in step 2500. In step 2600, the client 112 monitors for messages that can be received from the network 100. In step 2700, the client 112 provides information to enable the manager to perform the assessment steps described above.

Similarly, in step 2800, the client 112 monitors his or her conditions, as appropriate, and provides the results of the monitoring to the network for evaluation by the manager. The client 112 also performs self-evaluation activities in step 2900. In step 3000, the client 112 receives, reviews and uses the educational information provided by the manager, which assists the client 112 with his or her self-management activities in step 3100. Clients may seek educational information independently as well. The client 112 also receives the heart-flash information in step 3200, and performs health sub-portal access activities in step 3300.

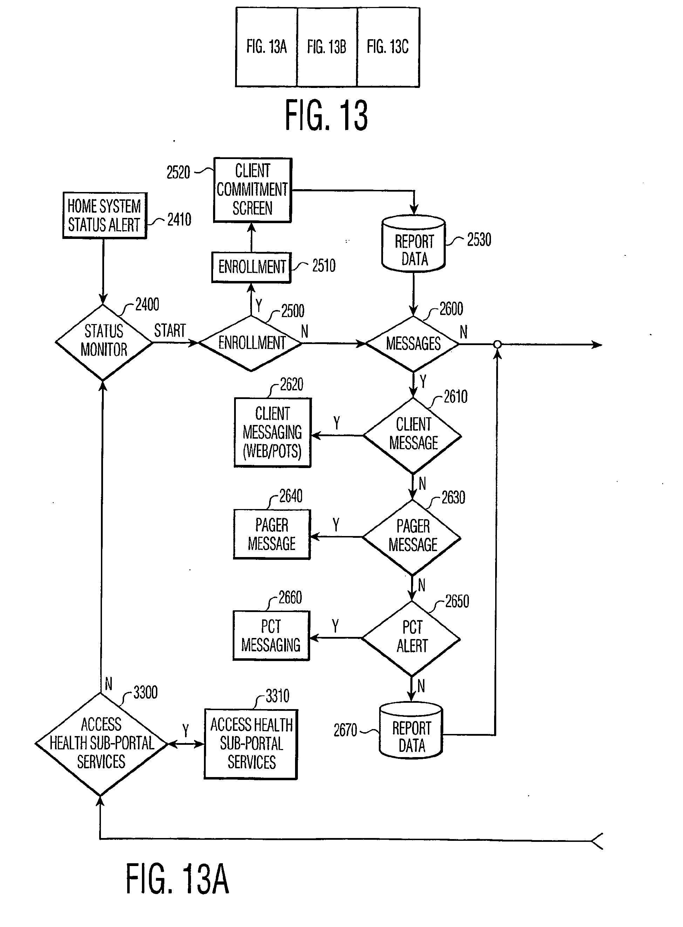

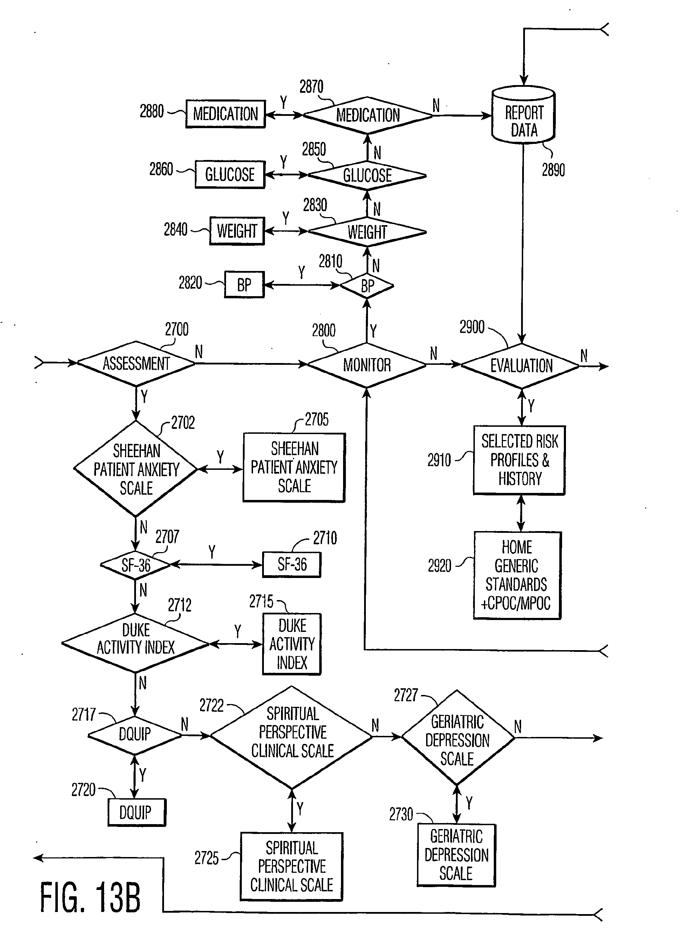

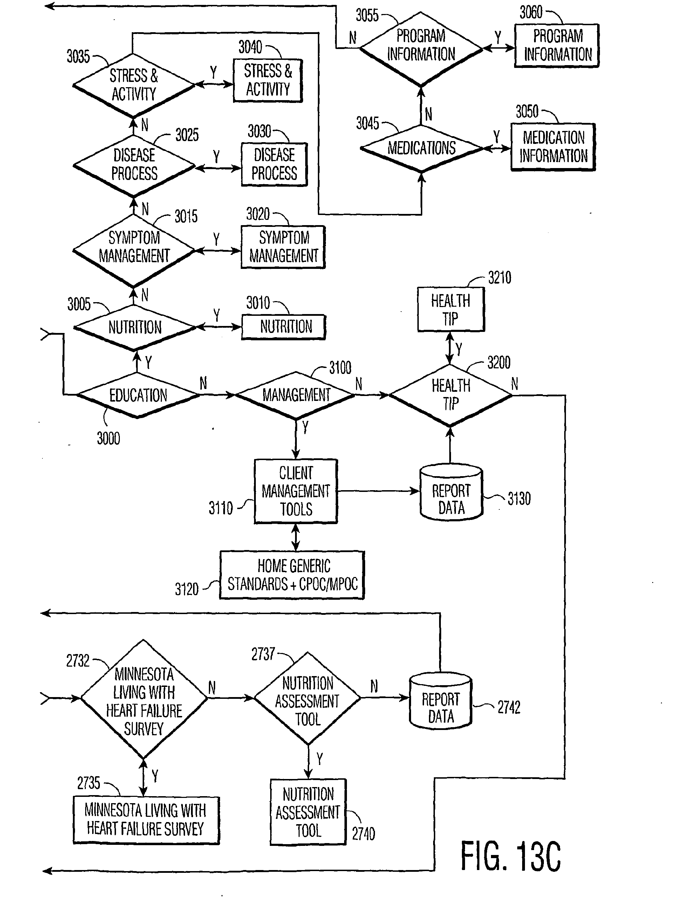

Further details of the steps shown in Fig. 12 will now be illustrated and described with respect to the flowchart in Fig. 13. As discussed above, the client 112 awaits receipt of a status alert in step 2400. When the network 100 provides a status alert to the client 112 in step 2410, the client 112 determines in step 2500 whether any enrollment activity needs to be performed. If enrollment activities are to be performed, the client 112 performs the enrollment activities described above in step 2510. Also, in step 2520, the client 112 can interact with a commitment screen which is displayed on the client workstation 114 (see Fig. 1), to report data to the centralized network 102 in step 2530. The centralized network 102 can store the data in the appropriate format in data base 104.

Once the enrollment activity has either been completed or skipped, as appropriate, the client 112 can determine in step 2600 whether he or she has received any messages. For example, in steps 2610, 2630 and 2650, the client 112 determines whether he or she has received a client message, a page, or a PCT alert, respectively, and takes the appropriate action in steps 2620, 2640 and 2660, respectively. The client 112 then reports the data to the centralized network 102 in step 2670, which can store the data in data base 104 and allow the data to be accessed by the appropriate personnel.

Once the messaging activity has been completed or skipped, as appropriate, assessment activities may be performed. If so, the client 112 participates in steps 2702, 2707, 2712 and 2717 to complete any medical history updates, SF-36 forms, Duke Activity Indexes or DQIPs, respectively. Generally, the manager determines the schedule for monitoring and assessments. However, the client may also initiate these processes as desired. If any of these tools need to be used to assess the client's status, the client 112 uses the appropriate tools in steps 2705, 2710, 2715 and 2720, as necessary, and then continues to step 2722.

In steps 2722, 2727, 2732 and 2737, any spiritual perspective clinical scales, geriatric depression scales, Minnesota Living with Heart Failure Surveys, or nutrition assessment tools, respectively, may be used. Generally, the manager determines the schedule for monitoring and assessments. However, the client may also initiate these processes as desired. If any of these tools need to be used to assess the client's status, the client 112 uses the appropriate tools in steps 2725, 2730, 2735 and 2740, as necessary, and then continues to step 2742. In step 2742, the client 112 reports the data obtained from the use of all the tools described above to the centralized network 102, which stores the relevant data in the centralized data base 104. The processing then proceeds to step 2800.

In step 2800, the client 112 uses the appropriate tools for monitoring and assessing his or her status, if necessary. For example, the client 112 or manager will determine whether the client's blood pressure needs to be monitored in step 2810. If so, the client 112 will take his or her blood pressure reading in step 2020. The client 112 or manager may also determine in steps 2830, 2850 and 2870 whether the client's weight, glucose measurement and medication, respectively, need to be monitored and will take the weight measurement, glucose measurement and medication in steps 2840, 2860 and 2880 respectively, as necessary. Once the appropriate measurements have been taken, the client 112 reports the data in step 2890 to the centralized network 102.

The client's activity then proceeds to step 2900, where the client 112 or manager determines whether or not to perform a self-evaluation. If a self evaluation is to be performed, the client 112 uses selected profiles and history information in step 2910, as well as generic standards for CPOC and MPOC, to perform the self evaluation.

The client's activity then proceeds to determine in step 3000 whether the education activity should be performed. If so, the client 112 or manager determine in steps 3005, 3015, 3025, 3035, 3045 and 3055 whether information relating to the client's nutrition, symptom management, disease process, stress and activity, medications and treatment program, respectively, should be received from the client's health manager. If any of this information should be received, the client 112 receives and uses the information in steps 3010, 3020, 3030, 3040, 3050 and 3060, respectively, as appropriate. In step 2890, the client 112 then reports any data obtained in the above steps to the centralized network 102, which can store the data in the centralized data base 104 as necessary. The network education tools may assess the client's understanding of the educational material and customize further sessions to optimize learning of important material. These tools may be interactive and delivered over high capacity data networks.

The client 112 then determines in step 3100 whether he or she needs to perform self management. If so, the client 112 in step 3110 uses the client management tools provided by the manager, along with the generic standards for CPOC and MPOC in step 3120. The health manager may grant permission to the client to engage in self-management processes contingent on successful completion of education and assessment programs. In step 3130, the client 112 then reports any data obtained in the above steps to the centralized network 102, which can store the data in the centralized data base 104 as necessary.

If the manager has sent a health tip to the client 112, the client 112 can receive the health tip in steps 3200 and review the information in step 3210. In step 3300, the client 112 determines whether to access health sub-portal services. If so, the client 112 accesses the services in step 3310. After the above steps have been either performed or skipped, as appropriate, the client 112 awaits receipt of another status alert in step 2400, and the above steps are repeated as necessary.

Although Figs. 12 and 13 show the steps in a particular order, certain steps can be performed simultaneously, and the steps can otherwise be performed in any practical order which will achieve the intended outcome.

As can be appreciated from the above, the network 100 and corresponding business model shown in Fig. 2 presupposes a minimal set of services and technology required to achieve critical mass for effective client management. Since all stakeholders find value, they are all motivated to participate in the program. Although conventional disease management services provide high intensity clinical support for patients at relatively high cost, information technology can significantly reduce the cost of delivering high intensity services. For instance, Web technology and the Internet permits asynchronous communications between clients 112 and individual providers. Since the provider does not have to be online at the same time as the client 112 (as in a video arrangement), the provider can prioritize interventions based on real importance rather than urgency.

Further, this environment provides significant improvements through automation. Traditional disease management enterprises use paper records or require manual entry of assessment data into a database. However, the network 100 uses automated entry of client data into a database where it can be processed and presented to the providers in a format which maximizes their productivity. This automation includes patient stratification and surveillance using provider-defined criteria. In the event of emergency, the network 100 can escalate priority to notify the provider in real time thus limiting liability associated with higher data flux. Also, as discussed above, the payors can access and collect the data for clients who are the payor's responsibility. The payors can then statistically review the collected data to determine, for example, whether any client's should receive reimbursements, and for outcomes analysis, preapproval of claims, and so on.

Furthermore, state of the art disease management provides specialized tools for patient education. Clients will find answers to chronic problems on the Web that will help them feel better. Also, as discussed above, inexpensive devices (blood pressure, scale, glucometer, thermometer, therapeutic devices, diagnostic instruments) can be connected to an information appliance (for example an interactive TV) which serves as a conduit for automated transfer of this information over a data network to the provider's server for processing and presentation to the providers. This information can, in turn, be transferred to a second database for outcomes analysis and benchmarking within the health delivery organization. This information can be processed to remove patient identities and analyzed for intersystem benchmarking and outcome comparisons.

As discussed in the background section above, there have been few disease management studies that credibly demonstrate attractive clinical and economic outcomes. Clinical outcomes should be based on easily measured results such as hospitalizations or emergency visits. Numerous tools exist to evaluate quality of life including SF-36, the Minnesota Living with Heart Failure survey and others. Economic outcomes require detailed information about interventions and their cost. These studies need to be performed at sufficient scale and duration such that the results are statistically valid. Only when these studies are replicated at a number of diverse healthcare organizations do the results become truly credible.

The network 100 described above can establish the necessary credibility. First, credibility is established by bringing together multiple medical centers experienced in large clinical trials. Second, IDNs afford the best location for these studies since they have access to financial information on diverse patient populations in multiple care delivery venues. Certain at-risk IDNs are unique in having both world class reputation and the broad diversity to evaluate clinical and economic outcomes as described above.

The network 100 provides a third way to ensure credibility by engaging a well-known independent actuary to establish the credibility of the results. The network 100 further enhances the credibility of the disease management system by gaining access to more information about patient status using remote monitoring and assessment. A high degree of credibility will enable simple business arrangements based on per patient per month charges rather than difficult to administer, less profitable risk sharing agreements. As explained above, the network 100 provides software tools, devices, protocols, computers, and data networks resident at the local provider sites.

In addition, local providers are installed, customized, and trained, so that they will, in turn, provide care to local patients. Local providers will have an option for local branding or co-branding with a world class medical center. Since care is delivered locally, credibility with local individual providers and provider organizations is likely to be high versus remote management. Further, the network 100 provides a venue for converting expensive acute care operations to more profitable and sustainable preventive operations. This is especially important given the number of local disease management start-ups that fail due to lack of expertise, resources needed to achieve scale, insufficient value, or inability to demonstrate credible outcomes.

The network 100 further provides for an effective use of information technologies to facilitate behavior modification through rapid promulgation of best medical practices, standardization and quality control of medical best practices, customization to individual patient (client 112) needs, and specialized patient education and self-management tools. As explained above, specialized devices can transmit data from remote sites that is extremely useful for rapid assessment of patient status. These assessments are almost invisible to the patient. The resulting early interventions enhance the patient's wellness and motivation to participate in the management process.

Patients will also participate in the management process, and they may self-manage their conditions once they achieve proficiency with disease states. For example, a patient may obtain on-line help with insomnia associated with medication schedules. Behavioral assessment tools such as "Rodger's Readiness to Change" can profile a patient's readiness and ability to participate in a management process before funds are committed.

Care providers often know what behaviors are desirable, but have difficulty convincing patients to adopt healthy behaviors. On-line help for providers will suggest important motivational techniques to assist with these situations.

It can be further appreciated that control of disease management protocols and rapid evaluation facilitated by telecommunications are keys to driving the measurement and evaluation cycle. Patients managed under the network 100 will benefit from the most progressive evidenced-based data driven paradigms. It is well known that rapid responses to client questions are very important to building confidence in a service organization. Telecommunications can provide these services in a unique and cost-effective venue. New developments in medical practice may be rapidly promulgated through these networks. Protocols may be standardized across systems where appropriate. Appropriate latitude can be granted to accommodate both individual patient goals and requirements and the special needs of local populations. Also, the patient's primary care physician will have control over the patient's care.

Although only a few exemplary embodiments of this invention have been described in detail above, those skilled in the art will readily appreciate that many modifications are possible in the exemplary embodiments without materially departing from the novel teachings and advantages of this invention. Accordingly, all such modifications are intended to be included within the scope of this invention.

## Claims

1. A system for monitoring health-related conditions of patients, comprising:
a plurality of remote monitoring stations (114), each being adapted to receive patient health-related data pertaining to a respective patient; and
a computer network (100) comprising a database containing accumulated health-related data pertaining to health-related conditions and treatment, and at least one data access device, adapted to provide a health care provider access to said computer network and said database, said computer network (100) being adapted to receive said patient health-related data from said remote monitoring stations (114);
said remote monitoring station (114) being adapted to receive from a respective patient said patient health-related data relating to the integration of a selected one of said treatment programs into the patient's lifestyle, the data comprising at least one of questions concerning health or treatment and responses to questions concerning health or treatment, that are generated using said remote monitoring station (114);
said computer network (100) being configured with assessment tools to allow a health care provider to assess said patient health-related data to determine progress of the patient on the selected treatment program and whether information relating to the selected treatment program needs to be conveyed to the patient;
**characterised in that** said computer network (100) is further adapted to establish treatment program for said patient based on their respective patient health-related data and said accumulated health-related data, and to revise said accumulated health-related data based on said patient health-related data.

2. A system as claimed in claim 1, wherein:
each of said remote monitoring stations comprises at least one measuring device, adapted to measure a physiological condition of said respective patient, and to provide data representative of said physiological condition for inclusion among said patient health-related data; and
said assessment tools allow a health care provider to monitor said patient health-related data relating to integration of a selected one of said treatment programs into the patient's lifestyle and determine readiness of the patient for self-management under the selected treatment program.

3. A system as claimed in claim 1 wherein:
said remote monitoring stations are adapted to provide said patient health-related data to said computer network over the Internet.

4. A system as claimed in claim 1 wherein:
said assessment tools are selected from the group consisting of Standard Form-36 (SF-36), Duke Activity Index, guidelines of the Diabetes Quality Improvement Project (DQIP), tools for specific disease state monitoring, depression scales, nutrition assessment tools, quality of life assessment tools

5. A system as claimed in claim 1, wherein:
said computer network is adapted to generate reports each including health-related information pertaining to a respective said patient.

6. A system as claimed in claim 1, wherein:
said computer network is adapted to provide said accumulated health-related data stored in said database to organizations financing at least a portion of said treatment programs, and is adapted to receive financial data pertaining to said treatment programs from said organizations and to store said financial data in said database.

7. A system as claimed in claim 1, wherein:
each said remote monitoring station receives from its respective said patient said health-related data including pertaining to the cardiovascular system of said patient.

## Patentansprüche

1. System zur Überwachung gesundheitsbezogener Zustände von Patienten, umfassend:
eine Vielzahl von Fernüberwachungsstationen (114), die jeweils dafür eingerichtet sind, patientengesundheitsbezogene Daten, die einen jeweiligen Patienten betreffen, zu empfanden; und
ein Computernetzwerk (100), umfassend eine Datenbasis, die angesammelte gesundheitsbezogene Daten enthält, die gesundheitsbezogene Zustände und Behandlung betreffen, und zumindest eine Datenzugangsvorrichtung, die dafür eingerichtet ist, einem Gesundheitsfürsorgeleistungserbringer Zugang zu dem Computernetzwerk und der Datenbasis zu ermöglichen, wobei das Computernetzwerk (100) dafür eingerichtet ist, die patientengesundheitsbezogenen Daten von den Fernüberwachungsstationen (114) zu empfangen;
wobei die Fernüberwachungsstation (114) dafür eingerichtet ist, von einem jeweiligen Patienten die patientengesundheitsbezogenen Daten zu empfangen, die sich auf die Integration eines ausgewählten der Behandlungsprogramme in den Lebensstil eines Patienten beziehen, wobei die Daten zumindest eines umfassen, nämlich Fragen hinsichtlich Gesundheit oder Behandlung und Antworten auf Fragen hinsichtlich Gesundheit oder Behandlung, die unter Verwendung der Fernüberwachungsstation (114) erzeugt werden;
wobei das Computernetzwerk (100) mit Bewertungswerkzeugen konfiguriert ist, um zu erlauben, dass ein Gesundheitsfürsorgeleistungserbringer die patientengesundheitsbezogenen Daten bewertet, um den Fortschritt des Patienten in dem ausgewählten Behandlungsprogramm zu bestimmen und zu bestimmen, ob Information, die sich auf das ausgewählte Behandlungsprogramm bezieht, an den Patienten übermittelt werden muss;
**dadurch gekennzeichnet, dass** das Computernetzwerk (100) ferner dafür eingerichtet ist, ein Behandlungsprogramm für den Patienten auf der Grundlage seiner jeweiligen patientengesundheitsbezogenen Daten und der angesammelten gesundheitsbezogenen Daten aufzustellen und die angesammelten gesundheitsbezogenen Daten auf der Grundlage der patientengesundheitsbezogenen Daten zur überprüfen,

2. System nach Anspruch 1, wobei:
jede der Fernüberwachungsstationen zumindest eine Messvorrichtung umfasst, die dafür eingerichtet ist, einen physiologischen Zustand des jeweiligen Patienten zu messen und Daten, die den physiologischen Zustand darstellen, zur Einbeziehung in die patientengesundheitsbezogenen Daten bereitzustellen; und
wobei die Bewertungswerkzeuge erlauben, dass ein Gesundheitsfürsorgeleistungserbringer die patientengesundheitsbezogenen Daten, die sich auf die Integration eines ausgewählten der Behandlungsprogramme in den Lebensstil des Patienten beziehen, überwacht und die Bereitschaft des Patienten zur Selbstverwaltung unter dem ausgewählten Behandlungsprogramm bestimmt.

3. System nach Anspruch 1, wobei:
die Fernüberwachungsstationen dafür eingerichtet sind, die patientengesundheitsbezogenen Daten über das Internet an das Computernetzwerk zu übergeben.

4. System nach Anspruch 1, wobei:
die Bewertungswerkzeuge aus der Gruppe ausgewählt werden, die aus Folgendem besteht: Standard Form-36 (SF-36), Duke Activity Index, Richtlinien für das Diabetes Quality Improvement Project (DQIP), Werkzeuge für spezifische Krankheitszustandsüberwachung Depressionsskalen, Ernährungsbewertungswerkzeuge, Lebensqualitätsbewertungswerkzeuge.

5. System nach Anspruch 1, wobei:
das Computernetzwerk dafür eingerichtet ist, Meldungen zu erzeugen, die jeweils gesundheitsbezogene Information, die einen jeweiligen Patienten betrifft, aufweisen.

6. System nach Anspruch 1, wobei:
das Computernetzwerk dafür eingerichtet ist, die angesammelten gesundheitsbezogenen Daten, die in der Datenbasis gespeichert sind, an Organisationen zu übergeben, die zumindest einen Teil der Behandlungsprogramme finanzieren, und dafür eingerichtet ist, Finanzdaten, die die Behandlungsprogramme betreffen, von den Organisationen zu empfangen und die Finanzdaten in der Datenbasis zu speichern.

7. System nach Anspruch 1, wobei:
jede Fernüberwachungsstation von ihrem jeweiligen Patienten die gesundheitsbezogenen Daten empfängt, einschließlich derjenigen die das kardiovaskuläre System des Patienten betreffen.

## Revendications

1. Système pour surveiller des états liés à la santé de patients, comportant :
une pluralité de stations de surveillance à distance (114), qui sont chacune aptes à recevoir des données liées à la santé d'un patient concernant un patient respectif; et
un réseau informatique (100) comportant une base de données contenant des données accumulées liées à la santé relatives à un traitement et à des états liés à la santé, et au moins un dispositif d'accès aux données, apte à délivrer un accès à un fournisseur de soins de santé audit réseau informatique et à ladite base de données, ledit réseau informatique (100) étant apte à recevoir lesdites données liées à la santé d'un patient en provenance de ladite station de surveillance à distance (114) ;
ladite station de surveillance à distance (114) étant apte à recevoir d'un patient respectif lesdites données liées à la santé d'un patient relatives à l'intégration d'un programme de traitement sélectionné parmi lesdits programmes de traitement dans le mode de vie du patient, les données comportant au moins l'une parmi des questions connexes à la santé ou au traitement et des réponses à des questions connexes à la santé ou au traitement qui sont générées en faisant appel à ladite station de surveillance à distance (114) ;
ledit réseau informatique (100) étant configuré avec des outils d'évaluation en vue de permettre à un fournisseur de soins de santé d'évaluer lesdites données liées à la santé d'un patient, afin de déterminer les progrès du patient dans le cadre du programme de traitement choisi et de déterminer si les informations relatives au programme de traitement choisi doivent être transmises au patient ;
**caractérisé en ce que** ledit réseau informatique (100) est en outre apte à établir un programme de traitement destiné audit patient sur la base des données respectives liées à la santé d'un patient et desdites données accumulées liées à la santé, et à réexaminer lesdites données accumulées liées à la santé sur la base desdites données liées à la santé d'un patient.

2. Système selon la revendication 1, dans lequel :
chacune desdites stations de surveillance à distance comporte au moins un dispositif de mesure, apte à mesurer un état psychologique dudit patient respectif, et à délivrer des données représentant ledit état psychologique destinées à intégrer lesdites données liées à la santé d'un patient ; et
lesdits outils d'évaluation permettent à un fournisseur de soins de santé de surveiller lesdites données liées à la santé d'un patient en relation à l'intégration d'un programme de traitement sélectionné parmi lesdits programmes de traitement dans le mode de vie du patient et de déterminer l'aptitude du patient à une gestion autonome en regard du programme de traitement sélectionné.

3. Système selon la revendication 1, dans lequel :
lesdites stations de surveillance à distance sont aptes à fournir lesdites données liées à la santé d'un patient audit réseau informatique par le biais d'Internet.

4. Système selon la revendication 1, dans lequel :
lesdits outils d'évaluation sont sélectionnés dans le groupe comportant un questionnaire généraliste « 36 » (SF-36), un index d'activité de Duke, des directives du projet d'amélioration de la qualité des diabètes (DQIP), des outils de surveillance d'état de pathologies spécifiques, des échelles de dépression, des outils d'évaluation de la nutrition, des outils d'évaluation de la qualité de vie.

5. Système selon la revendication 1, dans lequel :
ledit réseau informatique est apte à générer des rapports comportant chacun des informations liées à la santé concernant un patient respectif.

6. Système selon la revendication 1, dans lequel :
ledit réseau informatique est apte à délivrer lesdites données accumulées liées à la santé qui sont stockées dans la base données à des organisations finançant au moins une partie desdits programmes de traitement, et est apte à recevoir des données financières connexes auxdits programmes de traitement en provenance desdites organisations et à stocker lesdites données financières dans ladite base de données.

7. Système selon la revendication 1, dans lequel :
chaque station de surveillance à distance reçoit de son dit patient respectif lesdites données liées à la santé, y compris des données convexes au système cardiovasculaire dudit patient.
